# EUROPEAN PATENT SPECIFICATION

(11) **EP 4 267 042 B1**
(45) Date of publication and mention of the grant of the patent: **04.02.2026**
(21) Application number: 21836212.7
(22) Date of filing: 22.12.2021
(51) Int. Cl.: A61F 2/38, A61F 2/46, A61F 2/30

(54) **PROSTHETIC ELBOW JOINT IMPLANT AND KIT**
PROTHETISCHES ELLENBOGENGELENKIMPLANTAT UND KIT
IMPLANT PROTHÉTIQUE D'ARTICULATION DU COUDE ET KIT

(30) Priority: 22.12.2020 EP 20216523
(43) Date of publication of application: 01.11.2023
(73) Proprietor: Universiteit Gent, 9000 Gent (BE)
(72) Inventor: HERREGODTS, Jan, 9500 Geraardsbergen (BE); VAN TONGEL, Alexander, 9090 Melle (BE); DE WILDE, Lieven, 9000 Gent (BE); HERREGODTS, Stijn, 9500 Geraardsbergen (BE)
(74) Representative: De Clercq & Partners
(86) International application number: PCT/EP2021/087300
(87) International publication number: WO 2022/136550

(56) References cited:
- EP-A1- 0 575 107
- US-A1- 2006 111 788
- US-A1- 2006 111 789
- US-A1- 2019 269 517

## Description

### Field

The present invention is in a field of an elbow joint implant. It is also in a field of measurement tools for measurement of direction of the ulna in a subject.

### Background

A well described complication after total elbow arthroplasty is loosening of the components and polyethylene wear. Misalignment of the implant in the frontal plane is identified as a major cause of these complications. An aim of the presently-described device or kit is to reduce loosening of the components and polyethylene wear.

A problem of the art is how to measure variation in the ulna direction in a subject, in particular in the proximal ulna that avoids medical imaging. It is an aim to provide a measurement tool for determining a native direction of the ulna.

A further problem in the art in elbow arthroplasty is tendon-to-bone healing of the triceps. The triceps are often damaged or cut, depending on the procedure. The triceps-off approach reveals complication rates that are 4 times higher than the triceps-on approach. The main complication of triceps-off approach is triceps ruptures.

US 2019/0269517 A1 discloses an elbow prothesis having a stem and flange, wherein an opening of flange is directed superiorly to allow for the capture of tissue or bone when stem is slid into the intramedullary canal of the humerus during implantation. US 2006/0111788 discloses an elbow prosthesis including an ulnar component and a humeral component, wherein a second portion of the humeral component is rotatably connected to a first portion of said humeral component about a longitudinal axis. EP 0 575 107 discloses a joint prosthesis, for use in surgery, comprises two elongate members adapted to be fixed respectively to associated bones, e.g. to the humerus and to the ulna, the elongate members comprising complementary means for a hinged attachment at respective ends thereof.

An aim of the presently described device or kit is to provide a prosthetic elbow joint implant that facilitates a triceps-on approach.

### Summary of some embodiments

Provided herein in accordance with claim 1 is an elbow joint prosthetic implant (100) comprising:
- a humeral component (50) comprising a humeral stem portion (60) configured for placement within the humeral intramedullary canal of a subject
- an ulnar component (110) comprising an ulnar stem portion (120) configured for placement within the ulnar intramedullary canal of the subject,
wherein the humeral component (50) and ulnar component (110) are connected by an implant elbow joint (130),
- wherein the ulnar component (110) further comprises an angulation joint (140) having at least one degree of freedom of rotational movement configured to adjust a direction of the ulnar stem portion (120). The angulation joint (140) is separate from the implant elbow joint (130). The angulation joint (140) is further configured to fix a varus-valgus direction of the ulnar stem portion (120).

The angulation joint (140) may have only one degree of freedom of rotational movement, and is configured to adjust and fix a direction of the ulnar stem portion (120) to correspond with a native varus-valgus direction of the ulna (200) of the subject.

The angulation joint (140) may have two or three degrees of freedom of rotational movement, and the implant (100) is configured to adjust and fix a direction of the ulnar stem portion (120) to correspond with a native direction of the ulna (200) of a subject.

The implant elbow joint (130) may comprise a proximal part (130a) configured to couple to a distal part (130b) thereby forming the implant elbow joint (130); the angulation joint (140) may comprise a proximal angulation coupling (140a) configured to couple to a distal angulation coupling (140b), thereby forming the angulation joint (140); the ulnar component (110) may comprise a linking body (146) having a proximal end (10) and a distal end (20); the proximal end (10) of the linking body (146) may be disposed with the distal part (130b) of the elbow joint (130); the distal end (20) of the linking body (146) may be disposed with the proximal part (140b) of the angulation joint (140); the position and/or orientation of the distal elbow coupling (130b) and the proximal angulation coupling (140a) may be fixed, and the linking body (146) may be dismountable from the ulnar component (110).

The elbow joint prosthetic implant (100) may be provided with a plurality of linking bodies (146), at least two, preferably all having a different fixed position and/or different fixed orientation relation between the elbow joint distal part (130b) and the proximal angulation coupling (140a), preferably wherein at least two preferably all of plurality of linking bodies (146) has a different fixed distance (h) between the elbow joint distal part (130b) and the proximal angulation coupling (140a).

The elbow joint prosthetic implant (100) may be provided with:
- a locking element (148) configured for locking a direction and/or axial rotational angle of the ulnar stem portion (120),
- a fixing element (148') configured for fixing the linking body (146) to the remainder of the ulnar stem portion (120), or
- a combined locking and fixing element (148, 148) configured for both locking a direction and/or axial rotational angle of the ulnar stem portion (120) and fixing the linking body (146) to the remainder of the ulnar stem portion (120).

The proximal angulation coupling (140a) and distal angulation coupling (140b) may form the angulation joint (140) have one degree of rotational freedom, one of the proximal angulation coupling (140a) or distal angulation coupling (140b) may comprise a tapered member (142a), the other of the proximal angulation coupling (140a) or distal angulation coupling (140b) may comprise a reciprocating tapered hole (142b) configured to engage the tapered member (142a) thereby forming the one degree of rotational freedom angulation joint (140).

The proximal angulation coupling (140a) and distal angulation coupling (140b) form the angulation joint (140) having two or three degrees of rotational freedom, one of the proximal angulation coupling (140a) or distal angulation coupling (140b) comprises a spherical member (144a), the other of proximal angulation coupling (140a) or distal angulation coupling (140b) comprises a reciprocating socket (144b) configured to engage the spherical member (144a), thereby forming the two or three degrees of rotational freedom angulation joint (140).

The ulnar stem portion may be a rigid monoblock. The ulnar stem portion (120) may be at least partially articulated, allowing the ulnar stem portion (120) to conform to the shape of the ulnar intramedullary canal (210).

Further provided is a kit (500) comprising: a humeral component (50) comprising a humeral stem portion (60) configured for placement within the humeral intramedullary canal of a subject, at least two ulnar components (110, 110') that are each monoblocks (*i.e.* each ulnar component (110, 110') is a monoblock), wherein each ulnar component (110, 110') comprises: an ulnar stem portion (120), configured for placement within the ulnar intramedullary canal (210) of the subject, a distal part (130b, 130b') of an (implant) elbow joint (130), configured to engage with a corresponding proximal part (130a) of the (implant) elbow joint (130) of the humeral component (50), thereby forming the (implant) elbow joint (130), and wherein at least two of the ulnar components (110, 110') mutually differ in a valgus-varus direction of the ulnar stem portion (120). At least one of the ulnar components (110, 110') may have a valgus angle of between -1° and 4° or between 9° and 29°. At least one ulnar component (110, 110') may have a valgus angle between 9° and 29°, preferably between 10° and 20°, more preferably between 12° and 18°.

The kit may be configured to provide an elbow joint prosthetic implant (100) with a selected ulnar component (110, 110') from the at least two ulnar components (110, 110') that restores a native varus-valgus direction of the ulna (200) of the subject.

Further provided is a measurement tool (1100), not according to the claimed invention, for determining a direction of an ulna (200) of a subject, the measurement tool comprising: a tool humeral component (150) comprising a tool humeral stem portion (160) configured for dismountable placement within the humeral intramedullary canal of the subject, and a tool ulnar component (1110) comprising an ulnar stem portion (1120) configured for dismountable placement within the ulnar intramedullary canal (210) of the subject, wherein the tool humeral component (150) and tool ulnar component (1110) are connected by a tool elbow joint (1130), the tool ulnar component (1110) further comprises a (separate) tool angulation joint (1140) configured to adjust a direction (preferably a varus-valgus direction) of the tool ulnar stem portion (1120), and one or more angles adopted by tool angulation joint (1140) is readable.

Further provided is a kit, not according to the claimed invention, comprising: a humeral component (50) comprising a humeral stem portion (60) configured for placement within the humeral intramedullary canal of a subject, an ulnar component (110) that is a monoblock comprising an ulnar stem portion (120) configured for placement within the ulnar intramedullary canal (210) of the subject, and a distal part (130b) of an implant elbow joint (130) configured to engage with a corresponding proximal part (130a) of the humeral component (50) of the elbow joint (130), at least two exchangeable liner components (132, 132'): each and every one being configured for dismountable attachment to the distal part (130b, 130b') of the elbow joint (130), or each and every one being configured for dismountable attachment to the proximal part (130a) of the elbow joint (130), and each and every one being configured set and fix a different direction of the implant (100) elbow joint (130) axis of rotation (A-A') relative to the direction of the ulnar stem portion (120). Each and every exchangeable liner component may set a different varus-valgus direction of the ulna (200) of the subject. Each and every exchangeable liner component may set a different valgus angle of the ulna (200) of the subject. At least one sets the ulnar stem portion (120), in particular the proximal ulnar stem (120a), to an valgus angle between -1° and 4° or between 9° and 29°.

Not currently claimed is an elbow joint prosthetic implant (100) comprising:
- a humeral component (50) comprising a humeral stem portion (60) configured for placement within the humeral intramedullary canal of a subject,
- an ulnar component (110, 110') that is a monoblock comprising:
   ∘ an ulnar stem portion (120) configured for placement within the ulnar intramedullary canal (210) of the subject, and
   ∘ a distal part (130b, 130b') of an elbow joint (130), configured to engage with a corresponding proximal part (130a) of the elbow joint (130) of the humeral component (50), thereby forming the elbow joint (130),
wherein the ulnar component (110, 110') has a valgus angle of between 9° and 29°, preferably between 10° and 20°, more preferably between 12° and 18°.

### Figure Legends

**FIG. 1** is a schematic illustration of an elbow joint implant as described herein.
**FIG. 2A** is a medial-lateral view of an exemplary a left elbow joint implant as described herein.
**FIG. 2B** is a posterior-anterior view of the elbow joint implant of FIG. 2A.
**FIG. 2C** shows directional labelling applied to the orientation of the implant of FIG. 2B.
**FIG. 3** shows directional labelling used herein applied to a person stood upright and looking out from the page.
**FIG. 4A** and **4B** shows a view of implant of FIG. 2A with the humeral component (FIG. 4A) and ulnar component (FIG. 4B) separated; the distal part of the implant elbow joint is exemplarily depicted as a ring.
**FIG. 4C** and **4D** shows a view of implant of FIG. 2A with the humeral component (FIG. 4A) and ulnar component (FIG. 4B) separated; the distal part of the implant elbow joint is exemplarily depicted as a ring arc.
**FIG. 5** shows a view of implant of FIG. 2A wherein the angulation joint is a 1DOF revolute joint.
**FIG. 6** shows a view of implant of FIG. 2A wherein the angulation joint is a 2 or 3DOF ball joint.
**FIG. 7A** shows a view of implant of FIG. 2B wherein the native direction of the ulna matches the direction of the ulnar component.
**FIG. 7B** shows a view of implant of FIG. 2B wherein the native direction of the ulna is different from the example of FIG. 7A, and the direction of the ulnar component used in FIG. 7A would not restore the native direction of the ulnar component.
**FIG. 7C** shows directional labelling applied to the orientation of the ulna and to implant of **FIG. 8** is a schematic view of a linking body.
**FIG. 9A** to **C** are schematic views of 3 linking bodies each with a different distance (h), and wherein the angulation joint is a 1DOF joint.
**FIG. 10A** to **C** are schematic views of 3 linking bodies each with a different distance (h), and wherein the angulation joint is a 2DOF or 3DOF joint.

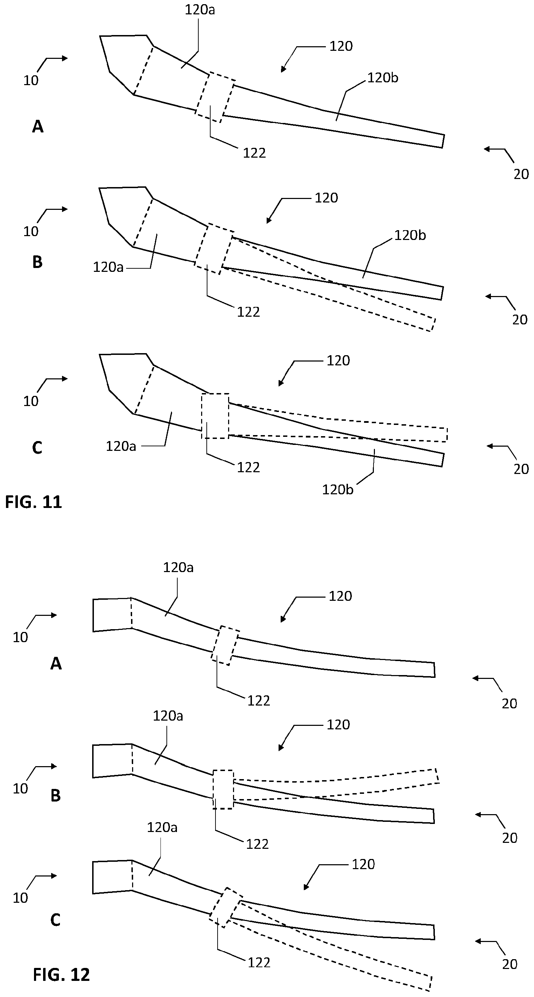

**FIG. 11A** to **C** are schematic medial-lateral views of an ulnar component shown in FIG. 2A each provided with an articulation joint each set at different directions.
**FIG. 12A** to **C** are schematic posterior-anterior views of an ulnar component in FIG. 2B each provided with an articulation joint each set at different directions, corresponding to
**FIGs. 13A** to **13C** are isometric views of an implant as described here. A left elbow implant is shown in medial-lateral view (A), anterior posterior- view (B), posterior-anterior view (C).

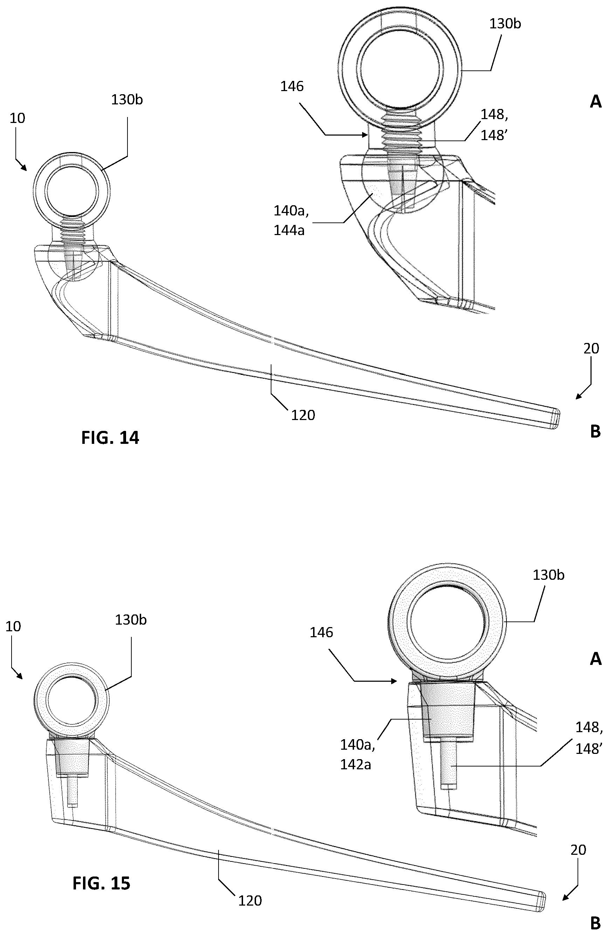

**FIGs. 14A** to **14B** are isometric views of an implant as described here provided with a linking body having a 2- or 3-DOF angulation joint.
**FIGs. 15A** to **15B** are isometric views of an implant as described here provided with a linking body having a 1-DOF angulation joint.
**FIG.16** is view of an ulna and intramedullary canal.
**FIG.16A** shows directional labelling applied to the orientation of the ulna of FIG. 16.
**FIG.17A** shows a projection of the 1^{st} and 2^{nd} linear lines onto a measurement plane.
**FIG.17B** shows a projection of the 1st linear line and flexion-extension axis (a-a') of rotation onto a measurement plane.
**FIG.18** is view of an ulnar component of FIG. 2B, proximal ulnar stem, distal ulnar stem, and best fit lines.
**FIG.19** is view of an ulnar component of FIG. 18 with 1st linear line and axis of rotation (A-A') of the implant elbow joint indicated.
**FIG.19A** shows directional labelling applied to the orientation of the ulna of FIG. 18 and 19.
**FIG. 20A** shows a projection of the 1st linear line and 2^{nd} linear onto a measurement plane
**FIG. 20B** shows projection of the 1st linear line and implant elbow joint axis (A-A') of rotation onto a measurement plane.
**FIG. 21** shows a kit containing two elbow joint implants each having a different direction of the ulnar stem portion. The implant is based on FIG. 2B but without the angulation joint.
**FIG. 22A** and **B** show different views of a measurement tool (not according to the claimed invention). The view is based on FIGs. 2A and 2B.
**FIG. 23A** and **B** show different views of an implant provided with a fixation element receiver. The views are based on FIGs. 2A and 2B.
**FIG. 23C** and **D** show different views of an implant positioned within the ulna provided with a fixation element receiver.
**FIG. 24** show views from a study of the imaged ulnas with their proximal joints aligned.
**FIG. 24A** shows directional labelling applied to the orientation of the ulnas of FIG. 24.
**FIG. 25** is an isometric view of an implant ulnar component disposed with an exchangeable liner component (not according to the claimed invention).
**FIG. 25A** is a detail of FIG. 25 showing a part of the exchangeable liner component (not according to the claimed invention).
**FIG. 26** is an isometric view an exchangeable liner component (not according to the claimed invention).
**FIG. 27** is an isometric view an exchangeable liner component, separated in two portions (not according to the claimed invention).
**FIG. 28** are views (**A** and **B**) of two different exchangeable components, each giving the ulnar stem portion a different direction (not according to the claimed invention).
**FIG. 29** is an isometric view of a humeral component having a humeral angulation joint.
**FIG. 30A** is a transparent wireframe view of a distal part of a humeral component having the proximal humeral angulation coupling.
**FIG. 30B** is an isometric view of a humeral linking body (76).
**FIG. 31A** and **B** are isometric views of a humeral linking body (76) wherein the distal humeral angulation coupling (70b) has different lateral offsets.
**FIG. 32A** and **B** are isometric views of a humeral linking body (76) wherein the distal humeral angulation coupling (70b) has different anteroposterior offsets.
**FIG. 33A** and **B** are isometric views of a humeral linking body (76) wherein the distal humeral angulation coupling (70b) has different superoinferior offsets.
**FIG. 34** is a views of a humeral measurement tool (700) for measurement of the direction of the humerus (not according to the claimed invention).

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.,* any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The following detailed description, therefore, is not to be taken in a limiting sense, and the scope of the present invention is defined by the appended claims.

The terms "distal" and "proximal" are used through the specification to refer to a direction of the ulna or implant, and are terms generally understood in the field. "Proximal" means towards the shoulder and thus away from the hand. Conversely, "distal" means towards the hand and, therefore, away from the shoulder.

The "subject" refers to a person receiving the implant or measurement tool. The "user" refers to a person or persons applying the implant or measurement tool. The user may be a surgeon, in particular orthopedic surgeon, clinician, physician, or medical assistant.

The elbow joint is a term known in the art and is a joint of a subject rotating around a flexion-extension axis (a-a'), and connects the ulna (200) to the humerus. The rotation of the elbow joint around the flexion-extension axis (a-a') refers to the rotation of the ulnohumeral joint. As is well understood in the art, there is an angulation of the flexion-extension axis (a-a'), to the neutral position of the humerus, so in resting position the axis may not be not in the frontal plane. The flexion-extension axis hence may not be perpendicular to the anatomical humeral shaft (neither in frontal plane nor in axial plane).

The varus (Vr) direction is a term known in the art, and refers to an inward direction, this is towards the subject body's midline. The direction is at least medial and may be in plane that is the frontal plane or parallel thereto. With respect to the ulna it is an inward (medial) direction in relation to the humerus when the elbow joint is in extension. It may be referenced to the flexion-extension axis (a-a').

The valgus (VI) direction is a term known in the art, and refers to an outward direction, this is away the subject body's midline. The direction is at least lateral and may in plane that is the frontal plane or parallel thereto. With respect to the ulna it is an outward (lateral) direction in relation to the humerus when the elbow joint is in extension. It may be referenced to the flexion-extension axis (a-a').

The varus-valgus (Vr-VI) direction refers to a direction that may be a valgus or varus.

The frontal plane or coronal plane are terms known in the art and it divides the body into dorsal and ventral (back and front, or posterior and anterior) portions.

The anterior (A) direction is a term known in the art, and refers to a front (face) direction of the subject. With respect to the ulna it is a forward direction in relation to the humerus when the elbow joint is in extension. It may be referenced to the flexion-extension axis (a-a').

The posterior (P) direction is a term known in the art, and refers to a rear direction of the subject. With respect to the ulna it is a rearward direction in relation to the humerus when the elbow joint is in extension. It may be referenced to the flexion-extension axis (a-a'). The lateral (L) direction is a term known in the art, and refers to a sideways (left to right, or vice versa) direction of the subject. With respect to the ulna it is a sideways direction in relation to the humerus when the elbow joint is in extension. It may be referenced to the flexion-extension axis (a-a').

The superior (S) direction is a term known in the art, and refers to an upward (towards the head) direction of the subject. With respect to the ulna it is towards the humerus when the elbow joint is in extension. It may be referenced to the flexion-extension axis (a-a').

The inferior (I) direction is a term known in the art, and refers to a downward (towards the feet) direction of the subject. With respect to the ulna it is towards the hand when the elbow joint is in extension. It may be referenced to the flexion-extension axis (a-a').

The medial direction is a term known in the art, and means toward the midline of the body. With respect to the ulna it is a sideways direction in relation to the humerus when the elbow joint is in extension. It may be referenced to the flexion-extension axis (a-a').

The cranial direction is a term known in the art, and refers to an upward (towards the head) direction of the subject. With respect to the ulna it is towards the humerus when the elbow joint is in extension. It may be referenced to the flexion-extension axis (a-a').

The caudal direction is a term known in the art, and refers to a downward (towards the feet) direction of the subject. With respect to the ulna it is towards the hand when the elbow joint is in extension. It may be referenced to the flexion-extension axis (a-a').

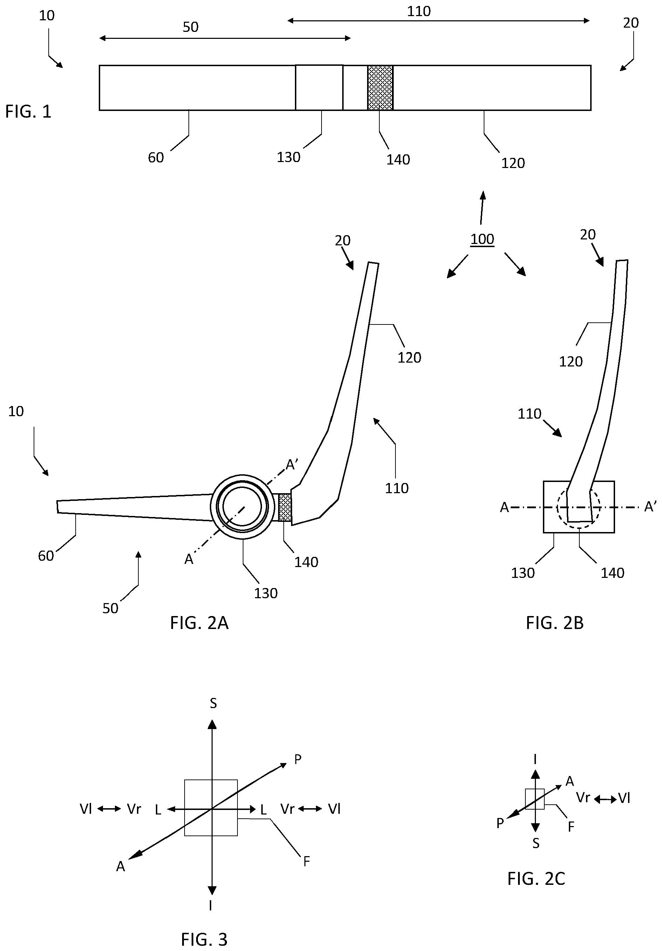

**FIG.** 3 shows directional labelling used herein superior (S), inferior (I), posterior (P), anterior (A), valgus (VI), varus (Vr), lateral (L), frontal (F) applied to a person stood upright and looking out from the page.

The native direction of the ulna (200) of the subject refers to the direction of the ulnar canal in one or more of varus, valgus, anterior, posterior directions, in particular to a direction of the proximal ulnar canal. It is referenced to the flexion-extension axis (a-a') of rotation of the subject elbow joint. The term native refers to the ulna direction prior to implantation and giving rise to a normal functioning of the elbow joint. The implant having the angulation joint (140) adjusts the implant so providing post-operatively the ulna with the native direction.

The native varus-valgus direction of the ulna (200) of the subject refers to the direction of the ulna in a varus or valgus direction, in particular of the proximal ulnar canal. It is referenced to flexion-extension axis (a-a') of rotation of the subject elbow joint. The term native refers to the direction prior to implantation and giving rise to a normal functioning of the elbow joint. The implant having the angulation joint (140) adjusts the implant so providing post-operatively the ulna with the native varus-valgus direction.

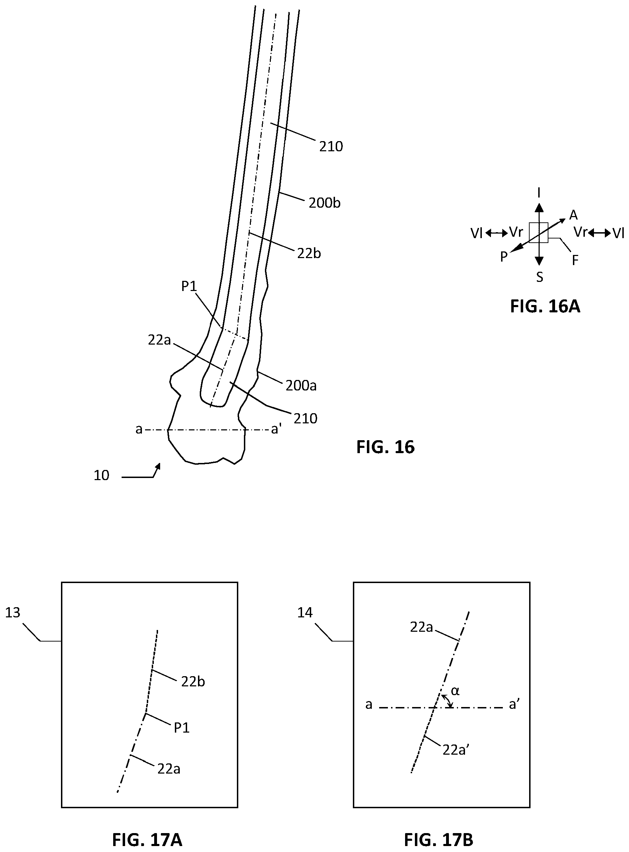

The proximal ulna (200a) refers to a proximal part of the ulna (200) of the subject located between the flexion-extension axis (a-a') of rotation of the subject elbow joint and a point where a direction of the ulnar intramedullary canal (210) (also referred to as "canal" herein) changes in a varus-valgus direction **(P1).** Typically, the proximal ulna (200a) occupies between 20% to 40% of the length of the total length of the ulnar. The proximal ulna (200a) is shown in **FIG. 16****.**

The distal ulna (200b) refers to a distal part of the ulna (200) adjacent distally to the proximal ulna (200a) of the subject. It is located distal of the point where the direction of the intramedullary canal changes in the varus-valgus direction **(P1).** Typically, the distal ulna (200b) occupies between 60% to 80% of the length of the total length of the ulnar. The distal ulna (200b) is partially shown in **FIG. 16****.**

The present invention relates to an elbow joint prosthetic implant (100) or a kit that takes account of the valgus angulation variation in the subject.

The present inventors have found that the native valgus angle of the proximal ulna has a large variation in the population. This is proven by an anatomical study showing a range of valgus angulation between 1.33° and 16.07° (alpha (see below) between 73.93° and 88.67°) in the included population which has been found for the first time. Since manufacturers generally only offer one standard implant, the implant does not correctly restore the anatomical axis of rotation in across the population. Valgus-varus malrotation is the main contributor in early implant wear and loosening.

The present invention provides an elbow implant (100) or kit that provides different configurations of elbow implant (100) that restores the native varus-valgus direction of the ulna (200), in particular of the proximal ulna of the subject. By being configurable, the implant or kit addresses the problems caused by the wide variance of valgus angulation in the population. A schematic elbow joint prosthetic implant (100) is shown in **FIG. 1****.**

Provided herein is an elbow joint prosthetic implant (100) comprising:
- a humeral component (50) comprising a humeral stem portion (60) configured for placement within the humeral intramedullary canal of a subject,
- an ulnar component (110) comprising an ulnar stem portion (120) configured for placement within the ulnar intramedullary canal of the subject,
wherein the humeral component (50) and ulnar component (110) are connected by an implant elbow joint (130),
- wherein the ulnar component (110) further comprises an angulation joint (140) having at least one degree of freedom of rotational movement configured to adjust a direction of the ulnar stem portion (120).

Exemplary elbow joint prosthetic implants (100) provided with an angulation joint (140) are shown in **FIGs. 2A, 2B****,** **4A to 4C****,** **5, 6****,** **7A, 7B****,** **13A-C****,** **23A, 23B****.**

The implant elbow joint (130) is a joint of the implant (100) replacing the elbow joint of the subject. The implant elbow joint (130) of the implant (100) is typically a hinge joint rotating around an axis of rotation (A-A'). The axis of rotation (A-A') is typically parallel to the flexion-extension axis (a-a') of rotation of the subject elbow joint. It may or may be co-axial with the flexion-extension axis (a-a') of rotation of the subject elbow joint. In some cases, the user will want to reproduce the position of the elbow joint, and the implant elbow joint (130) axis of rotation (A-A') will be co-axial with the elbow flexion-extension axis (a-a') of the subject. In other cases, the user will want to create a new axis of rotation to compensate for deficits, and the implant elbow joint (130) axis of rotation (A-A') will not be co-axial with the elbow flexion-extension axis (a-a') of the subject. The implant elbow joint (130) may be made from any biocompatible material having the requisite strength characteristic for an elbow joint prosthetic implant (100), such as one or more of titanium, stainless steel, tantalum, vanadium, cobalt, chromium, tungsten, nickel, molybdenum.

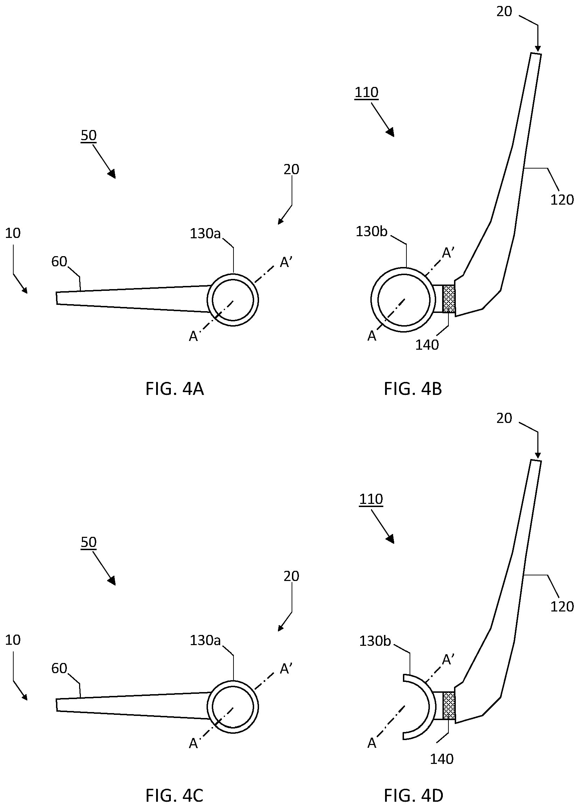

The implant elbow joint (130) connects the humeral component (50) and ulnar component (110). It typically comprises two parts that couple together to form the implant elbow joint (130). More specifically, the humeral component (50) comprises a proximal part (130a) of the implant elbow joint (130), and the ulnar component (110) comprises a distal part (130b) of the implant elbow joint (130). Coupling of the proximal part (130a) and distal part (130b) forms the implant elbow joint (130). More specifically, the implant elbow joint (130) may comprise a proximal part (130a) configured for coupling to the distal part (130b) thereby forming the implant elbow joint (130). The respective elbow parts (130a, 130b) are shown in **FIGs. 4A** to **4C.** The humeral stem portion (60) extends in a proximal direction from the proximal part (130a) of the joint (130). The humeral stem portion (60) and proximal part (130a) of the joint are preferably rigidly and non-adjustably mutually attached. The ulnar stem portion (120) extends in a distal direction from the distal part (130b) of the joint (130). The ulnar stem portion (120) and distal part (130b) of the joint (130) are connected by the angulation joint (140). The proximal part (130a) and distal part (130b) of the implant elbow joint (130) may be shaped for coupling to each other, for instance as a ring, cylindrical member, concave surface, sphere, semi-sphere. They may be mutually complementary in shape.

The implant elbow joint (130) may be fully- constrained or semi-constrained. The fully constrained implant elbow joint (130) of the implant (100) is typically a hinge joint that rotates around the axis of rotation (A-A') without joint laxity. The semi-constrained implant elbow joint (130) is hinge joint that rotates around an axis of rotation (A-A'), where the joint exhibits laxity. Laxity is toggle play in the joint that minimises the effect of forces transmitted to the bone/cement interface as the center of the axis of rotation changes during flexion and extension. The joint laxity may be in any direction; preferably it is in a varus-valgus direction. The joint laxity may in a range of 2 to 10°; most preferably it is a valgus laxity (*e.g.* 1 to 5° range) and/or varus laxity (*e.g.* 1 to 5° range). The direction of the axis of rotation (A-A') may be at a mid-point of the range of laxity.

The distal part (130b) and proximal part (130a) of the implant elbow joint (130) may be coupled to each other and attached by a retainer (*e.g.* by a pin or retaining edges) to prevent separation of the parts (130a, 130b); the elbow joint prosthetic implant (100) is known as a linked implant. **FIGs. 4A** and **4B** show the distal part (130b) and proximal part (130a) of a linked implant. Alternatively, the distal part (130b) and proximal part (130a) of the implant elbow joint (130) may couple to each other without a retainer and the parts (130a, 130b) are maintained coupled by soft-tissue tension; the elbow joint prosthetic implant (100) is known as an unlinked implant. **FIGs. 4C** and **4D** show the distal part (130b) and proximal part (130a) of an unlinked implant. Alternatively, the elbow joint prosthetic implant (100) may be a semi-linked implant that can be configured in the linked configuration or in the unlinked configuration.

The implant elbow joint (130) may further comprise a liner component provided on the proximal part (130a), or on the distal part (130b), or both parts (130a, 130b). The liner component may be configured to reduce friction between the articulating surfaces of both parts (130a, 130b). The liner component may set a direction of the implant elbow joint axis (A-A') as described later below. The liner component may be made from a polymer such as polyethylene or silicone. The liner component may be non-detachable or be detachable.

The ulnar stem portion (120) is a part of the ulnar component (110) and is configured for placement within the humeral intramedullary canal of a subject. It is shaped essentially longitudinally. It is positioned distal of the angulation joint (140). It is configured to be oriented in a superior-inferior direction when the implant elbow joint (130) is in extension. The ulnar stem portion (120) is dimensioned for insertion into the ulnar intramedullary canal (210), in particular into a proximal insertion zone of the ulnar intramedullary canal. The proximal insertion zone refers to region of the ulnar intramedullary canal prepared by the user by removal of bone tissue (e.g. trabecular bone and marrow) starting from the proximal end of the ulna, for receiving the ulnar stem portion (120). The proximal insertion zone may be between 20 to 70% of the total length of the ulna, but may be longer. It may be between 75-190 mm long, but may be longer. Accordingly, the length of the ulnar stem portion (120) may be between 75-190 mm in length, but may be longer or shorter. The ulnar stem portion (120) can be longer, for instance, in revision cases the ulnar stem portion (120) may occupy the length ulnar intramedullary canal. The ulnar stem portion (120) may be dimensioned to occupy the intramedullary canal in only the proximal ulna (200a) of the subject. The stem portion (120) may be dimension to occupy the intramedullary canal at least in the proximal ulna (200a) of the subject. The stem portion (120) may be dimension to occupy the intramedullary the proximal ulna (200a) and at least a part of the distal ulna (200b) of the subject. The proximal ulna (200a) and distal ulna (200b) are illustrated in **FIG. 16** and described later in detail below. The ulnar stem portion (120) may be made from any biocompatible material having the requisite strength characteristic for an elbow joint prosthetic implant (100), such as titanium, stainless steel, tantalum, vanadium, cobalt, chromium, tungsten, nickel, molybdenum.

The humeral stem portion (60) is a part of the humeral component (50) extending in a proximal direction from the elbow joint (130). The humeral stem portion (60) is dimensioned for insertion into the humeral intramedullary canal, in particular into a humeral insertion zone of the humeral intramedullary canal. The humeral insertion zone refers to region of the humeral intramedullary canal prepared by the surgeon by removal of bone tissue (e.g. marrow) starting from the distal end of the humeral, for receiving the humeral stem portion (60). It is typically between 20 to 75% of the total length of the humerus. It is typically between 70-250mm long Accordingly, the length of the humeral stem portion (60) may be between 100-200 mm in length The humeral stem portion (60) can be longer, for instance, in revision cases the humeral stem portion (60) may occupy the length humeral intramedullary canal. The humeral stem portion (60) may be made from any biocompatible material having the requisite strength characteristic for an elbow joint prosthetic implant (100), such as titanium, stainless steel, tantalum, vanadium, cobalt, chromium, tungsten, nickel, molybdenum.

The angulation joint (140) is a mechanical rotational joint having at least 1DOF of rotational movement (*e.g.* 1, 2 or 3 DOF). It is also known as an ulnar angulation joint (140). It is separate from the implant elbow joint (130). The angulation joint (140) may be configured to adjust (and fix) a varus-valgus direction of the ulnar stem portion (120). It may be configured to adjust (and fix) a direction of the ulnar stem portion (120) to correspond at least with a native varus-valgus direction of the ulna (200) of the subject. Once the direction is locked or fixed, rotation of angulation joint (140) is prevented. The angulation joint (140) maintains the fixed or locked direction after implantation (*in situ*). The angulation joint (140) may comprise a proximal angulation coupling (140a) configured to couple to a distal angulation coupling (140b), thereby forming the angulation joint (140). The rotational direction of the angulation joint (140) is lockable *i.e.* rotation of the angulation joint (140) can be prevented (fixed). The elbow joint prosthetic implant (100) may be provided with a locking element (*e.g.* a fixation screw) (148) configured for locking a direction and/or axial rotational angle of the ulnar stem portion (120). The angulation joint (140) may be provided without a locking element *i.e.* the angulation joint may be free moving; the direction may be fixed during implantation by cement used to secure the ulnar component (110) within the ulnar intramedullary canal of the subject.

The direction of the ulnar stem portion (120) of the implant refers to a direction of the ulnar stem portion (120), in particular, of the proximal ulnar stem (120a) (see later below). The direction may be in one or more of varus (Vr), valgus (VI), anterior (A), posterior (P) directions. The direction may be in one or more of varus (Vr), valgus (VI), and optionally additionally in one or more of the anterior (A) and posterior (P) directions. Hence the ulnar stem portion (120) may be adjustable in the aforementioned direction. It is referenced to an axis of rotation (A-A') of the implant elbow joint (130).

The direction of the ulnar stem portion (120) may be a valgus-varus direction of the ulnar stem portion (120). The valgus-varus direction of the ulnar stem portion (120) of the implant refers to a direction in a varus, valgus direction (frontal plane), of the ulnar stem portion (120) in particular to a direction of the proximal ulnar stem (120a) in a varus, valgus direction (frontal plane). Hence the ulnar stem portion (120) may be adjustable in the aforementioned direction. It is referenced to an axis of rotation (A-A') of the implant elbow joint (130).

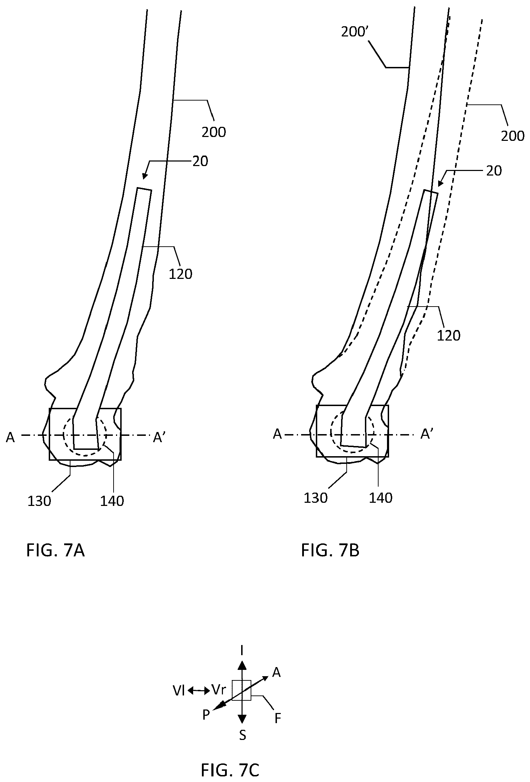

Prior or during implantation, the angulation joint (140) is adjusted and fixed so that the native varus-valgus direction of the ulna (200), in particular, of the proximal ulna (200a) of the subject is restored. The result is a restoration of native elbow kinematics, which reduces complications such as loosening and lining (*e.g.* polymer) wear. There is further a decrease in amount of revision surgery as loosening and wear are less frequent. There is further improved proprioception and stability after arthroplasty as the muscle tension between agonists and antagonists is in balance. The same implant can be applied to a wide population range. **FIGs 7A** and **7B** demonstrate two ulnas (200, 200') having different directions. **FIG. 7A** shows a view of an ulna stem portion (120) of the implant of that matches the native direction of the ulna (200). **FIG. 7B** shows a view of implant of **FIG. 7A** and the native direction of the ulna (200') is different from the example of **FIG. 7A** ((200) broken line), and the direction of the ulna stem portion (120) used in **FIG. 7A** no longer matches the ulna (200) and would not result in restoration of the native elbow kinematics, but would be restored by adjustment to the angulation joint (140).

It is appreciated that different sub-population have different sized bones. Accordingly, the elbow joint prosthetic implant (100) and parts therein may be provided in one or more different sizes to complement the size of the ulna or humerus in the subject.

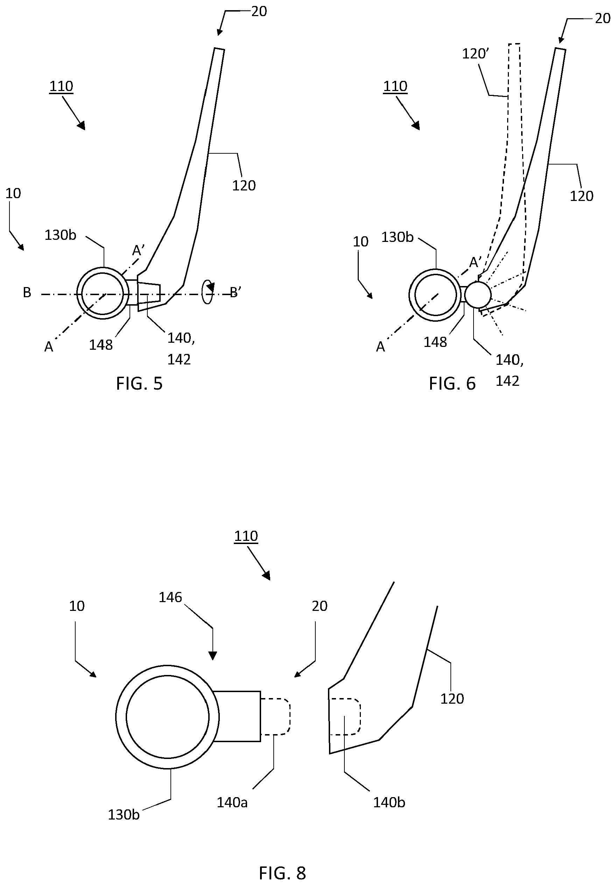

The angulation joint (140) may have only (exactly) 1DOF of rotational movement (around B-B'), as exemplified in **FIG. 5****.** Such implant (100) is configured to adjust a direction of the ulnar stem portion (120) to correspond with a native varus-valgus direction of the ulna (200) of the subject. The 1DOF of rotational movement of the angulation joint (140) has an axis of rotation (B-B') that may be perpendicular to an axis of rotation (A-A') of the implant elbow joint (130). The axis of rotation (B-B') may be perpendicular ± 0 to 15° in any direction to an axis of rotation (A-A') of the implant elbow joint (130). The direction of the angulation joint (140) axis of rotation (B-B') may deviate from being perpendicular to an axis of rotation (A-A') by a supero-/inferior-rotation (e.g. translation of the angulation joint (140) in more proximal / distal direction) in the sagittal plane. The axis of rotation (B-B') of the angulation joint (140) may or may not intersect the axis of rotation (A-A') of the implant elbow joint (130). The angulation joint (140) may allow rotation of at least 15°, preferably of at least 30°.

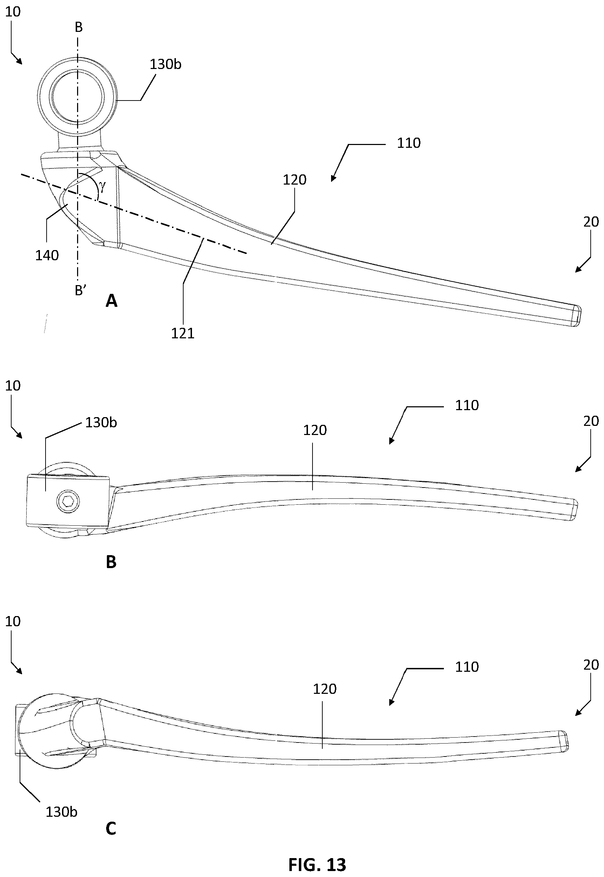

A best fit line (121) of the ulnar stem portion (120), in particular of the proximal ulnar stem (120a) may adopt an angle gamma with respect to the angulation joint (140) axis of rotation (B-B') when the implant is in a medial-lateral view (*e.g.* the view of FIG. 13A). The value of gamma may be 90 to 135°.

Examples of 1DOF of rotational movement is a revolute joint, in which the proximal angulation coupling (140a) and the distal angulation coupling (140b) are joined by fixture providing a single axis of rotation such as a pin, or nut and bolt or contains a machine taper.

The angulation joint (140) may have exactly two or exactly three degrees of freedom of (2DOF or 3DOF) rotational movement, as exemplified in **FIG. 6****.** Such the implant (100) may be configured to adjust and fix a direction of the ulnar stem portion (120) to correspond with a native direction of the ulna (200) of a subject. One of the degrees of freedom preferably includes the axis of rotation (B-B') of the angulation joint (140). The additional degrees of freedom allow movement in the anterior and/or posterior direction; additional rotation is clinically important. The 2DOF or 3DOF angulation joint (140) comprises a ball joint. Advantageously, 2DOF or 3DOF angulation joint (140) allow more directions for adjustment after placement; it is more convenient for the user to correct for imprecise implantation.

The range of motion of the 2DOF or 3DOF angulation joint (140) may be centred around the axis B-B', which corresponds to the 1DOF of rotational movement when the angulation joint (140) has only 1DOF.

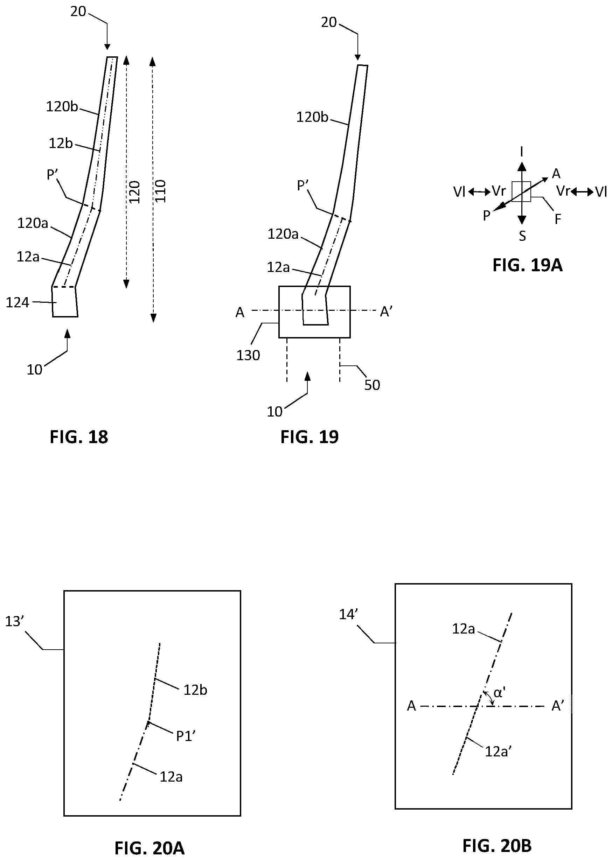

In order to orient the ulnar stem portion in the canal (120) in a superior-inferior direction and to restore the axis of rotation when the implant elbow joint (130) is in extension, a bridging section (124) is provided to introduce an angulation. The bridging section is highlighted in **FIG. 18****.** It is comprised in the ulnar component (110). It is typically continuous or a monoblock with the proximal ulnar stem (120a). It connects the ulnar stem portion (120) to the angulation joint (140). It connects the ulnar stem portion (120) to the implant elbow joint (130) when the angulation joint (140) is absent (see exchangeable liner component and kits). A best fit line of the bridging section and of the ulnar stem portion (120) or proximal ulnar stem (120a) may intersect at an angle (beta) of 90° to 150° degrees , preferably 100° to 135°.

The ulnar component (110) may comprise a linking body (146) having a proximal end (10) and a distal end (20). The linking body (146) is shown in exploded view **FIG. 8** The proximal end (10) of the linking body (146) is disposed with the elbow joint distal part (130b) described earlier. The distal end (20) of the linking body (146) is disposed with the proximal angulation coupling (140a) described earlier. A position and orientation of the elbow joint distal part (130b) with respect to the proximal angulation coupling (140a) is fixed. A plurality of linking bodies (146) may be provided, each having a different fixed position and/or fixed orientation between the elbow joint distal part (130b) and the proximal angulation coupling (140a).

The linking body (146) is dismountable from a remainder of ulnar component (110). In other words, the linking body (146) proximal angulation coupling (140a) is dismountable from the distal angulation coupling (140b). The linking body (146) is dismountable from a remainder of the implant (100). In other words, the linking body (146) proximal angulation coupling (140a) is dismountable from the distal angulation coupling (140b), and the linking body (146) elbow joint proximal part (130a) may be dismountable from the elbow joint distal part (130b).

A plurality of linking bodies (146) may be provided, at least two preferably all having a different fixed position and/or different fixed orientation relation between the elbow joint distal part (130b) and the proximal angulation coupling (140a). The dismountability allows a linking body (146) to be selected having a desired fixed position and/or fixed orientation relation between the elbow joint distal part (130b) and the proximal angulation coupling (140a). The plurality may be provided as a kit optionally with the implant.

A plurality of linking bodies (146) may be provided, at least two preferably all having a different fixed distance (h) between the elbow joint distal part (130b) and the proximal angulation coupling (140a). The fixed distance (h) provides different degrees of anterior-posterior translation. The dismountability allows a linking body (146) to be selected having a desired distance (h), allowing for a displacement adjustment in an anteroposterior direction. The plurality may be provided as a kit optionally with the implant.

A plurality of linking bodies (146) may be provided, at least two preferably all having a different fixed angle between the elbow joint distal part (130b) and the proximal angulation coupling (140a) is fixed. The dismountability allows a linking body (146) to be selected having a desired angulation, allowing for a displacement adjustment in a medio-lateral direction. The plurality may be provided as a kit optionally with the implant.

The dismountability may be before, during or after placement *in situ* of implant (100). For example, when both (humeral and ulnar) components are cemented in the bone, the user could still change the linking body (146) if the extension or flexion of the elbow still needs correcting. It also allows the linking body (146) to be changed in revision surgery.

The linking body (146) may be made from any biocompatible material having the requisite strength characteristic for an elbow joint prosthetic implant (100), such as one or more of titanium, stainless steel, tantalum, vanadium, cobalt, chromium, tungsten, nickel, molybdenum. The linking body (146) is preferably formed from a mono-block *i.e.* is a one-piece element. This provides adjustability to the implant while still maintaining mechanical strength by avoiding a prismatic joint.

A fixing element (*e.g.* threaded member) (148') may be provided configured for fixing the linking body (146) to the remainder of the ulnar stem portion (120) to prevent dismounting. The aforementioned locking element and fixing element may one and the same (e.g. threaded member).

Provided herein is a kit comprising:
- the humeral component (50),
- the ulnar component (110) comprising the angulation joint (140) and adapted for demountable attachment a linking body (146)
- a plurality of dismountable linking bodies (146), each having a different distance (h), and
- the implant elbow joint (130) connecting the humeral component (50) and ulnar component (110).

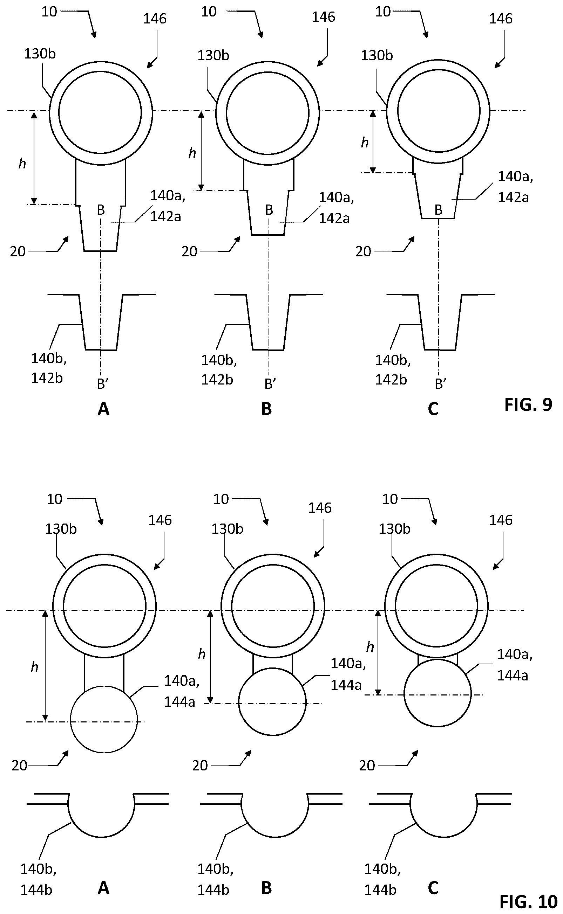

The removeable linking body (146) may be configured to provide the angulation joint (140) with one, preferably only one degree of rotational freedom (around B-B' axis). In other words, the proximal angulation coupling (140a) and distal angulation coupling (140b) forming the angulation joint (140) have one, preferably only one, degree of rotational freedom. One of the proximal angulation coupling (140a) or distal angulation coupling (140b) may comprise a tapered member (142a), and the other of the proximal angulation coupling (140a) or distal angulation coupling (140b) comprises a reciprocating tapered hole (142b) configured to engage the tapered member (142a) thereby forming the one degree of rotational freedom angulation joint (140). An example of a 1DOF removeable linking body (146) is shown in **FIGs. 9A** to **C****.** Each linking body (146) has a different fixed distance (h) between the elbow joint distal part (130b) and the proximal angulation coupling (140a).

The removeable linking body (146) may be configured to provide the angulation joint (140) with two or three, preferably only two or three, degrees of rotational freedom. In other words, the proximal angulation coupling (140a) and distal angulation coupling (140b) form the angulation joint (140) having two or three, preferably only two or three, degrees of rotational freedom. A combination of a rotation around the medio-lateral axis and another distance (h) allows a cranio-caudal adjustment. One of the proximal angulation coupling (140a) or distal angulation coupling (140b) may comprises a spherical member (144a), and the other of proximal angulation coupling (140a) or distal angulation coupling (140b) may comprises a reciprocating socket (144b) configured to engage the spherical member (144a), thereby forming the angulation joint (140). The spherical member (144a) may be part or all of a sphere. An example of a 2 or 3DOF removeable linking body (146) is shown in **FIGs. 10A** to **C****.** Each linking body (146) has a different fixed distance (h) between the elbow joint distal part (130b) and the proximal angulation coupling (140a).

The proximal ulna (200a) refers to a proximal part of the ulna (200) of the subject located between the flexion-extension axis (a-a') of rotation of the subject elbow joint and a point (P1) where a direction of the intramedullary canal (210) changes in the varus-valgus direction. Point **(P1)** may be determined visually by the user (e.g. surgeon; radiologist), or from a medical image. Typically, the proximal ulna (200a) occupies between 20% and 40% (*e.g.* 58 to 116 mm) of the total length of the ulnar. The distal ulna (200b) refers to a distal part of the ulna adjacent to point P1, and distal of the proximal ulna (200a). The distal ulna (200b) occupies the remainder after the proximal ulna (200a), and typically, is between 60% and 80% (*e.g.* 174 to 232 mm) of the total length of the ulnar. **FIG. 16** shows a view of the ulna (200) with proximal ulna (200a), distal ulna (200b), and point **(P1)** where a direction of the intramedullary canal (210) changes.

The point **P1** corresponds to a point of varus angulation of the ulnar intramedullary canal (210). The point of varus angulation **(P1)** of the ulnar intramedullary canal (210) may be determined by:
- fitting on a 3D medical image of the ulna (200):
   - a 1st linear line (22a) along the ulnar intramedullary canal (210) in the proximal ulna (200a);
   - a 2nd linear line (22b) along the along the ulnar intramedullary canal in the distal ulna (200b);
- projecting the 1st (22a) and 2nd (22b) linear lines onto a measurement plane (13) parallel to the frontal plane (F),
- obtaining the point of varus angulation (P1) where the 1st linear line and 2nd linear line intersect.

The varus angle of ulnar intramedullary canal is between the 1^{st} and 2^{nd} linear lines at point **P1.** **FIG. 17A** shows a projection of the 1st (22a) and 2nd (22b) linear lines onto the measurement plane (13) an intersection at **P1** that is point of varus angulation.

The valgus angle of the ulnar intramedullary canal (200) of the subject refers to an angle of measurement defining the proximal ulna intramedullary canal (200) in a varus-valgus direction. It is sometimes known in the art as the valgus angulation. It is referenced to a flexion-extension axis (a-a') of rotation of the subject elbow joint. It is determined with the elbow joint in extension. The valgus angle alpha of the ulna intramedullary canal is an example of a specific angle of measurement of the subject defining the proximal ulna in the varus-valgus direction.

The angle alpha may be determined by:
- fitting a 1st linear line (22a) along the ulnar intramedullary canal in the proximal ulna (200a);
- projecting the 1st linear line (22a) and flexion-extension axis (a-a') of rotation of the subject elbow joint onto a measurement plane (14) parallel to the frontal plane (F), and extending (22a') the 1^{st} linear line (22a) to intersect the flexion-extension axis (a-a') of the elbow joint;
- measuring the angle between the projected linear lines on the measurement plane, which is the angle alpha.

**FIG. 17B** shows a projection of the extended 1st linear line (22a, 20a') and flexion-extension axis (a-a') onto the measurement plane (13) and intersection at angle alpha. Angle alpha has been found by the inventors to vary widely in the population between ~69° and 83°.

As mentioned elsewhere, ulnar stem portion (120) is configured for placement within the ulnar intramedullary canal (200) of a subject. It is shaped to follow the shape of the ulnar intramedullary canal. As mentioned elsewhere the direction of the ulnar stem portion (120) refers to the direction of the ulnar stem portion (120) in at least a varus, valgus direction (frontal plane). It may refer, in particular, to the direction of the proximal ulnar stem (120a) which is the ulnar stem portion (120) or a part thereof dimensioned to occupy the intramedullary canal within the proximal ulna of the subject. The ulnar stem portion (120) may be short and occupy only a part of the proximal ulna intramedullary canal (200a), in which case the proximal ulnar stem (120a) will have the same length as the whole ulnar stem portion (120). In most cases, the ulnar stem portion (120) occupies both the proximal (200a) and at least a part of the distal (200b) ulna intramedullary canal . In such case, the proximal ulnar stem (120a) extends distally to a point **(P1')** where a direction of the ulnar stem portion (120) changes in a varus-valgus direction corresponding to the point of varus angulation (P1) in the canal (200). The length proximal ulnar stem (120a) may be in the range 55 to 110 mm. The distal ulnar stem (120b), where present, is a part of the stem portion (120) extending in a distal direction from point **P1',** is adjacent to the proximal ulnar stem (120a), and corresponding to the distal ulna (200b) in the subject. The length makes up the remainder of the ulna after the proximal ulnar stem (120a). The length may be in the range 165 to 220 mm.

The point **P1'** corresponds to a point of varus angulation of the ulnar stem portion (120) of the implant (100). The point of implant varus angulation **(P1')** may be determined by:
- fitting on a 3D medical image of the ulna (200):
   - a 1st linear line (12a) along the proximal ulnar stem (120a);
   - a 2nd linear line (12b) along the along the distal ulnar stem (120b);
- projecting the 1st (12a) and 2nd (12b) linear lines onto a measurement plane (13') parallel to the frontal plane (F),
- obtaining the point of varus angulation (P1') where the 1st linear line and 2nd linear line intersect.

**FIG. 18** shows a view of the ulnar stem portion (120) with proximal ulnar stem (120a), distal ulnar stem (120b), with 1^{st} linear line (12a) and 2^{nd} linear line added and point **(P1')** where a direction of the ulnar stem portion (120) changes. **FIG. 19** is similar to the view of the **FIG. 18** with the elbow joint axis of rotation (A-A') indicated. **FIG. 20A** shows a projection of the 1st (12a) and 2nd (12b) linear lines onto the measurement plane (13') an intersection at **P1'** corresponding to a point of varus angulation (P1) in the intramedullary canal (200).

The valgus angle of the ulnar stem portion (120) of the implant (100) refers to an angle of measurement defining a direction of the ulnar stem portion (120), in particular of the proximal ulnar stem (120a) in a varus-valgus direction. It is sometimes known in the art as the valgus angulation. The valgus angle of the ulnar stem portion (120) of the implant (100) is the angle of the proximal ulnar stem (120a) proximal of the point of varus angulation (P1'). The value of the (implant) valgus angle remains constant for the implant (100) when the ulnar stem portion (120) distal of the point of varus angulation (P1') adopts different directions. The valgus angle of the implant corresponds with the valgus angle of the proximal ulna intramedullary canal (200). The implant valgus angle may range from ~61 to 95° (e.g. ~65 to 95°, or ~61 to 91°).

It is referenced to the axis of rotation (A-A') of the implant elbow joint (130). It is determined with the implant elbow joint (130) in extension.

The valgus angle "alpha-prime (α')" of the ulnar stem portion (120) is an example of a specific angle of measurement of the ulnar stem portion (120) or of the proximal ulnar stem (120a) in a varus-valgus direction. The angle alpha prime may be determined by:
- fitting a 1^{st} linear line (12a) along the ulnar stem portion (120), in particular along the proximal ulnar stem (120a),
- projecting the 1^{st} linear line (12a) and the axis of rotation (A-A') of the implant elbow joint (130) onto a measurement plane (14) parallel to the frontal plane (F), and extending (12a') the 1^{st} linear line (12a) to intersect the axis of rotation (A-A') of the elbow joint (130);
- measuring the angle on the measurement plane between the projected linear lines, which is the angle alpha prime.

**FIG. 20A** shows a projection of the 1st linear line extension (12a, 12a') and axis of rotation (A-A') onto the measurement plane (14') and intersection at angle alpha. The value of alpha prime of the implant corresponds with the value alpha of the ulnar intermedullary canal (210). The value of alpha prime may range from ~61 to 95° (*e.g.* ~65 to 95°, or ~61 to 91°). The value of alpha prime and of the implant valgus angle are related by a difference of 90°.

The ulnar stem portion (120) may be a rigid monoblock. The rigid monoblock is a one-piece element. It is typically formed by a process such as moulding, milling from a solid block, or additive manufacturing. It is rigid, meaning that it does not exhibit flexibility. It may be a rigid monoblock with the bridging section (124).

The ulnar stem portion (120) may be at least partially articulated, allowing the ulnar stem portion (120) to conform to the shape of the ulnar intramedullary canal. The ulnar stem portion (120) may be disposed with one or more accommodation joints (122) providing articulation to the ulnar stem portion (120). The one or more accommodation joints (122) are configured to adjust a shape of the ulnar stem portion (120) to comply at least partially with a shape of the ulnar insertion channel. The one or more accommodation joints (122) may be lockable. The accommodation joint (122) may be provided with a locking element (*e.g.* a fixation screw) configured for locking a direction and/or axial rotational angle of the joint. The accommodation joint (122) may be provided without a locking element *i.e.* the joint may be free moving. The angles of the one or more accommodation joints (122) may be fixed during implantation by cement used to secure the ulnar component (110) within the ulnar intramedullary canal of the subject. An accommodation joint (122) may be a 1 DOF, or 2 DOF or 3DOF rotational joint. An accommodation joint (122) may be a prismatic joint.

At least one of the accommodation joints (122) may be disposed at a position corresponding to a point of varus angulation (P1) of the ulnar intramedullary canal (210). In such case, the accommodation joint (122) is a rotational joint, preferably a 1, 2 or 3 DOF rotational joint. The accommodation joint (122) may be positioned distal of the proximal ulnar stem (120a), connecting the proximal ulnar stem (120a) to distal ulnar stem (120b). The accommodation joint (122) may be position at point P1' in the implant, corresponding to point P1 in the subject.

At least one of the accommodation joints (122) may be disposed at a position corresponding to a PUDA angle. The PUDA angle is defined in the lateral plane in an anterior-posterior direction. The angle PUDA may be determined by:
- Fitting a 1^{st} line along the posterior border of the proximal ulna
- Fitting a 2^{nd} line along the posterior border of the distal ulna
- Projecting the 1^{st} and 2^{nd} line onto a measurement plane parallel to the sagittal plane.
- Measuring the angle on the measurement plane between the projected linear lines, which is the angle PUDA. The value of the angle PUDA range from 0° to 20°.

Dorsal angulation (PUDA) is present in all specimen. This is the angle between a line/plane, parallel to the posterior border of the distal ulna and another line/plane, tangential to the dorsal crest of the olecranon. The minimum dorsal angulation is 0° and the maximal measured angulation is 14°, with an average PUDA of 5.7°. There is a strong correlation between the angulation of left and right ulna (r=0.86). A difference between both sides exists: in men, the angulation in right and left ulna is 6.3° and 6.6°, respectively. Whereas in woman, it is 5.7° and 4.6°. This indicates a significant difference on the left side between both sexes. The mean distance between point of angulation and tip is significantly greater in men. The average distance in men is respectively 48 mm and 51 mm for the right and left side, while the average distance in women is respectively 42 and 44 mm. Another study reports a ratio between the length to the dorsal angle and the total length of the ulna that ranges between 0.20 and 0.31.

In a variation of the implant (100), instead of providing an ulnar component (110) comprising an angulation joint (100), the ulnar stem portion (120) is rigidly and non-adjustably attached to the complementary part (distal part (130b)) of the implant elbow joint (130). The ulnar component (110) is hence formed as monoblock. The monoblock is a one-piece element. It is typically formed by a process such as moulding, milling from a solid block, or additive manufacturing. It is rigid, meaning that it does not exhibit flexibility.

The ulnar component (110) that is a monoblock may have a valgus angle of between 9° and 29°, preferably 15°±5° (*e.g.* between 10° and 20°), more preferably 15°±3° (*e.g.* between 12° and 18°). The inventors have found that the mean value of the valgus angle in the studied population was around 15°. Accordingly, an ulnar component (110, 110') monoblock with an implant valgus angle within the range encompassing the mean provides a single best-fit solution to early implant wear and loosening.

The ulnar component (110) that is a monoblock may have an alpha prime value of between 61° and 81°, preferably 75°±5° (*e.g.* between 70° and 80°), more preferably 75°±3° (*e.g.* between 72° and 78°). The inventors have found that the mean value of the valgus angle in the studied population corresponded to a value of alpha prime of around 75°. Accordingly, an ulnar component (110, 110') monoblock with an alpha prime value encompassing the mean range provides a single best-fit solution to early implant wear and loosening.

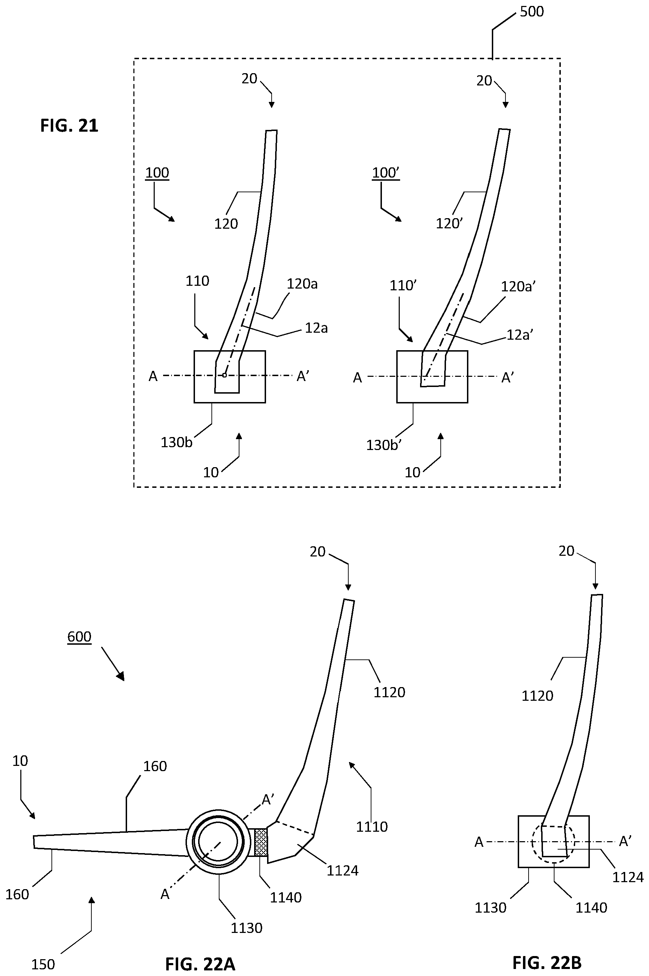

A plurality of ulnar component (110) monoblocks may be provided, each having a different direction of the ulnar stem portion (120) or of the proximal ulnar stem (120a), thereby replacing the angulation joint (140). The ulnar component (110) monoblock that restores the native varus-valgus direction of the ulna (200) of the subject is selected. **FIG. 21** depicts a kit (500) comprising two ulnar component (110, 110') monoblocks, each having a different direction of the ulnar stem portion (120, 120'), in particular of the proximal ulnar stem (120a, 120a'). A 1^{st} linear line (12a, 12b) fitted to the proximal ulnar stem (120a, 120a') is different between the monoblocks (120 and 120').

Provided herein is a kit (500) comprising:
- a humeral component (50) comprising a humeral stem portion (60) configured for placement within the humeral intramedullary canal of a subject, and a proximal part (130a) of an implant elbow joint (130)
- at least two (*e.g.* 2, 3, 4 ,5, or more) ulnar components (110, 110') that are each monoblocks, wherein each ulnar component (110, 110') comprises:
   ∘ an ulnar stem portion (120) configured for placement within the ulnar intramedullary canal (210) of the subject, and
   ∘ a distal part (130b, 130b') of an elbow joint (130), configured to engage with a corresponding proximal part (130a) of the elbow joint (130) of a humeral component (50), thereby forming the implant elbow joint (130),
wherein
at least two of the ulnar components (110, 110') mutually differ in a valgus-varus direction of the ulnar stem portion (120), and
the kit is configured to provide an elbow joint prosthetic implant (100) with a selected ulnar component (110, 110') from the at least two ulnar components (110, 110') that restores a native varus-valgus direction of the ulna (200) of the subject.

The kit may further comprise one or more humeral components (50), wherein each humeral component (50) comprises:
- a humeral stem portion (60) configured for placement within the humeral intramedullary canal, and
- the proximal part (130a) of the implant elbow joint (130).

At least one, preferably all of the ulnar components (110, 110') in the kit may have a valgus angle that is different from the others. The kit may comprise a plurality (*e.g.* 2, 3, 4, 5, or more) of ulnar components (110, 110'), each having a different valgus angle in a range between -1° to 29°.

The kit may comprise at least one ulnar component (110, 110') having a valgus angle between -1° and 4° or between 9° and 29°. The kit may comprise at least one ulnar component (110, 110') having a valgus angle between -1° and 3° or between 10° and 20°. The kit may comprise at least one ulnar component (110, 110') having a valgus angle between -1° and 2° or between 12° and 18°.

The kit may comprise at least one ulnar component (110, 110') having valgus angle of between 9° and 29°, preferably 15°±5° (*e.g.* between 10° and 20°), more preferably 15°±3° (*e.g.* between 12° and 18°). The inventors have found that the mean value of the valgus angle in the studied population was around 15°. Accordingly, an ulnar component (110, 110') monoblock with an implant valgus angle within the range encompassing the mean provides a single best-fit solution to early implant wear and loosening.

The kit may comprise at least two ulnar components (110, 110') each having a different valgus angle, one having a valgus angle of -1° to 4°, the other having a valgus angle of 9° to 22°. The kit may comprise at least three ulnar components (110, 110') each having a different valgus angle, one having a valgus angle of 19° to 29°, the other having valgus angle of 9° to 18°, and the other having a valgus angle of -1° to 8°.

At least one, preferably all of the ulnar components (110, 110') in the kit may have a value alpha prime that is different from the others. The kit may comprise a plurality (*e.g.* 2, 3, 4, 5, or more) of ulnar components (110, 110'), each having a different value of alpha prime in a range between 61° and 91°.

The kit may comprise at least one ulnar component (110, 110') having a value of alpha prime between 86° and 91° or between 61° and 81°. The kit may comprise at least one ulnar component (110, 110') having a value of alpha prime between 87° and 91° or between 70°and 80°. The kit may comprise at least one ulnar component (110, 110') having a value of alpha prime between 88° and 91° or between 72°and 78°.

The kit may comprise at least one ulnar component (110, 110') having a having a value of alpha prime between 61° and 81°, or 75°±5° (*e.g.* between 70° and 80°), or 75°±3° (*e.g.* between 72° and 78°). The inventors have found that the mean value of the valgus angle in the studied population corresponded to a value of alpha prime of around 75°. Accordingly, an ulnar component (110, 110') monoblock within with an alpha prime value encompassing the mean range provides a single best-fit solution to early implant wear and loosening.

The kit may comprise at least two ulnar components (110, 110') each having a different value of alpha prime, one having a value alpha prime 86°-91°, the other having a value alpha prime 68°-81°. The kit may comprise at least three ulnar components (110, 110') each having a different value of alpha prime, one having a value alpha prime of 61°-71°, the other having a value alpha prime of 71°-81°, and the other having a value alpha prime of 81-91°.

Each ulnar component (110) may be provided pre-attached to the humeral component (50) by the elbow joint (130). Each ulnar component may be provided not attached to the humeral component (50) by the elbow joint (130).

The above-mentioned parts of the kit, the humeral component (50) including humeral stem portion (60), ulnar component (110) (without the angulation joint (140)), ulnar stem portion (120), bridging section (130), elbow revolute joint (130) are as described elsewhere herein including constituting parts, components and variations.

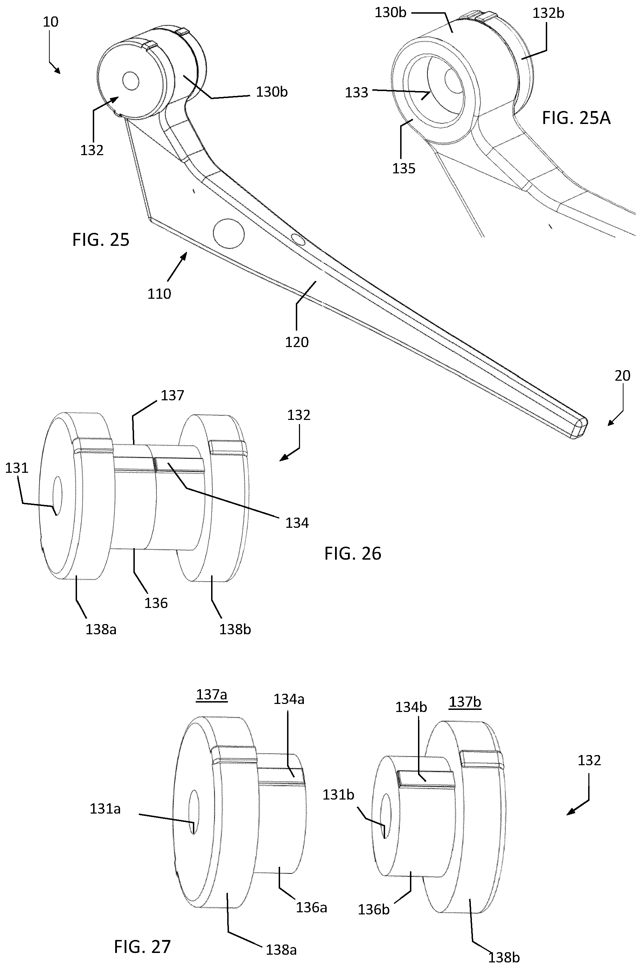

As mentioned earlier a liner component may be provided on the distal part (130b) of the implant elbow joint (130). The liner component may be an exchangeable liner component (132) configured to set and fix the direction of the elbow joint axis of rotation (A-A') relative to the direction of the ulnar stem portion (120), in particular of the proximal ulnar stem (120a). Illustrations of exemplary exchangeable liner component (132) are shown in **FIGs. 25** to **28****.** The exchangeable liner component (132) attaches to the distal part (130b) of the implant elbow joint (130) as shown in **FIGs. 25** and **25A****.**

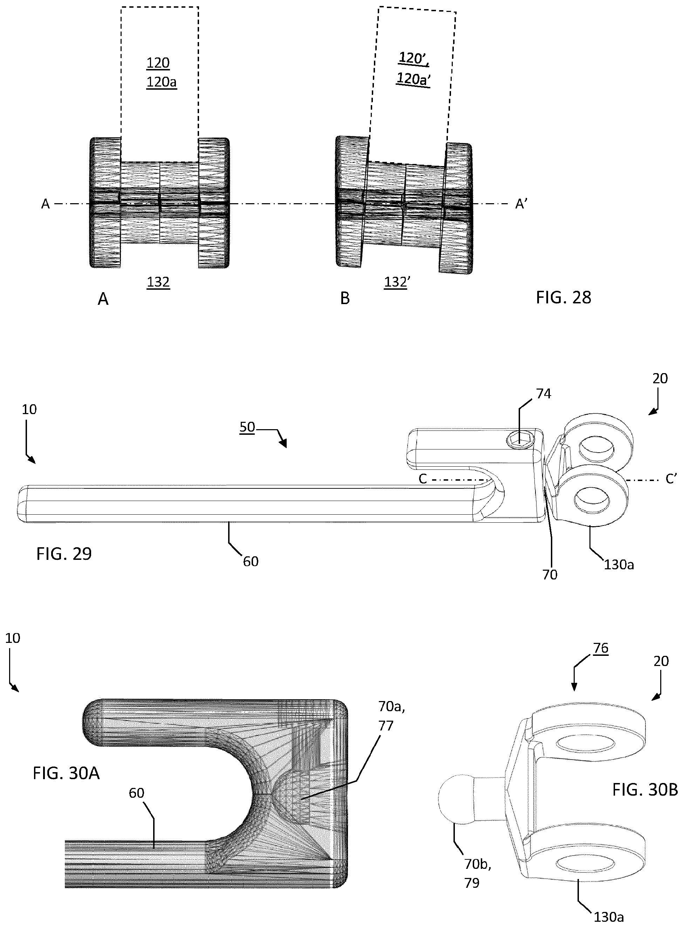

The direction of the ulnar stem portion (120), in particular, of the proximal ulnar stem (120a) may be set and fixed at different angles by swapping the exchangeable liner component (132) for one having a different direction of elbow joint axis of rotation (A-A'). Preferably, the exchangeable liner component (132) is used to adjust and fix a direction of the ulnar stem portion (120) in particular, of the proximal ulnar stem (120a) to correspond with the native varus-valgus direction of the ulna (200) of the subject. **FIG. 28A** and **28B** shows two exemplary exchangeable liner component (132, 132') each with a different direction of the ulnar stem portion (120) or the proximal ulnar stem (120a) relative to the elbow joint axis of rotation (A-A'). The difference in this example is around 7°, though it is appreciated that different values may be used. The elbow joint axis of rotation (A-A') may exhibit some degree of joint laxity (*e.g.* 2 to 8°) as described elsewhere herein.

The exchangeable liner component (132) is detachable from the distal part (130b) of the implant elbow joint (130). It may be secured by a fixation element, such as a bolt that secures the implant elbow joint (130). In some cases, fixation might not be necessary; the exchangeable liner component (132) may engage with the distal part (130b) of the implant elbow joint (130) and become fixed after reduction of the proximal (130a) and distal part (130b).

The function of the angulation joint (140) in adjusting the direction of the ulnar stem portion (120), in particular of the proximal ulnar stem (120a) is replaced by the exchangeable liner component (132). Hence, the angulation joint (140) is absent when the exchangeable liner component (132) is present.

The exchangeable liner component (132) may comprise a body (137) having a 1^{st} engaging part (136,138) that co-operates with the elbow joint distal part (130b). The cooperation with the 1^{st} engaging part (136,138) fixes the position and orientation of the exchangeable liner component (132) with respect to the elbow joint distal part (130b). Thus, the elbow joint axis of rotation (A-A') becomes fixed relative to the ulnar stem portion (120), in particular to the proximal ulnar stem (120a). The 1^{st} engaging part (136,138) may adopt one of a variety of different forms in order to fix its position and orientation with respect to the elbow joint distal part (130b).

In one example, the 1^{st} engaging part (136,138) may comprise a pair of flanges (138a, 138b) that flank the elbow joint distal part (130b), that increase contact surface area with the distal portion of the humeral component, and form the contact surface with the distal humeral joint component. They may also prevent sliding of the exchangeable component (132) along the elbow joint axis of rotation (A-A') relative to the distal part (130b) of the implant elbow joint (130). The flanges (138a, 138b) may abut side edges (135) of the elbow joint distal part (130b). The 1^{st} engaging part may comprise a rotation stop (134) that prevents rotation of the axis liner component (132) around the elbow joint axis of rotation (A-A') relative to the distal part (130b) of the implant elbow joint (130). The 1^{st} engaging part may comprise a core (136) that prevents other play relative to the distal part (130b) of the implant elbow joint (130). The core (136) may abut a rounded surface (133) of the distal part (130b) of the implant elbow joint (130).

The body (137) of the exchangeable liner component (132) may be provided with at least one 2^{nd} engaging part (131) that co-operates with the corresponding elbow joint proximal part (130a), and which guides rotation of the parts (130a, 130b) around the elbow joint axis of rotation (A-A').

The exchangeable liner component (132) body may be formed in two sections (137a, 137b). Two separated sections are shown in **FIG. 27****.** Each section (137a, 137b) may have a flange (138a, 138b). Each section (137a, 137b) may have a part of the core (136a, 136b). Each section (137a, 137b) may have a part of the rotation stop (134a, 134b).

Provided herein is a kit comprising:
- a humeral component (50) comprising a humeral stem portion (60) configured for placement within the humeral intramedullary canal of a subject, and a proximal part (130a) of an implant elbow joint (130);
- an ulnar component (110) that is a monoblock comprising an ulnar stem portion (120) configured for placement within the ulnar intramedullary canal (210) of the subject, and a distal part (130b) of the implant elbow joint (130);
- at least one (*e.g.* 2, 3, 4 ,5, or more) exchangeable liner component (132) configured for dismountable attachment to the distal part (130b, 130b') of the elbow joint (130).

The kit is configured to provide an elbow joint prosthetic implant (100) with a selected exchangeable liner component (132), from the at least two exchangeable liner components (132, 132'), that restores a native varus-valgus direction of the ulna (200) of the subject.

Preferably, the kit comprises at least two (*e.g.* 2,3,4,5, or more) exchangeable liner components (132, 132'), each configured to set and fix a different direction of the elbow joint axis of rotation (A-A') relative to the direction of the ulnar stem portion (120), in particular of the proximal ulnar stem (120a).

At least one, preferably all of the exchangeable liner components (132, 132') in the kit may set a different valgus angle that is different from the other exchangeable liner components (132, 132').

The kit may comprise a plurality (*e.g.* 2, 3, 4, 5, or more) of exchangeable liner components (132, 132'), each producing a different valgus angle in a range between -1° and 29°.

The kit may comprise at least one exchangeable liner component (132, 132') producing a valgus angle between -1° and 4° or between 9° and 29°. The kit may comprise at least one exchangeable liner components (132, 132') producing a valgus angle between -1° and 3° or between 10° and 20°. The kit may comprise at least one exchangeable liner components (132, 132') producing a valgus angle between -1° and 2° or between 12° and 18°.

The kit may comprise at least one exchangeable liner component (132, 132') producing a valgus angle of between 9° and 29°, preferably 15°±5° (*e.g.* between 10° and 20°), more preferably 15°±3° (e.g. between 12° and 18°). The inventors have found that the mean value of the valgus angle in the studied population was around 15°. Accordingly, the exchangeable liner component (132, 132') producing a valgus angle within the range encompassing the mean provides a single best-fit solution to early implant wear and loosening.

The kit may comprise at least two exchangeable liner components (132, 132') each producing a different valgus angle, one producing a valgus angle of -1° to 4°, the other producing a valgus angle 9° to 22°. The kit may comprise at least three exchangeable liner components (132, 132') each producing a different value of alpha prime, one producing a valgus angle 19° to 29°, the other producing a valgus angle of 9° to 19°, and the other producing a valgus angle -1° to 9°.

At least one, preferably all of the exchangeable liner components (132, 132') in the kit may induce a value alpha prime in the ulnar component (110) that is different from the other exchangeable liner components (132, 132').

The kit may comprise a plurality (*e.g.* 2, 3, 4, 5, or more) of exchangeable liner components (132, 132'), each producing a different value of alpha prime in a range between 61° and 91°.

The kit may comprise at least one exchangeable liner components (132, 132') producing a value of alpha prime of between 86° and 91° or between 61° and 81°. The kit may comprise at least one exchangeable liner components (132, 132') producing a value of alpha prime between 87° and 91° or between 70°and 80°. The kit may comprise at least one exchangeable liner component (132, 132') producing a value of alpha prime between 88° and 91° or between 72°and 78°.

The kit may comprise at least one exchangeable liner component (132, 132') producing a value of alpha prime of between 61° and 81°, preferably 75°±5° (*e.g.* between 70° and 80°), more preferably 75°±3° (*e.g.* between 72° and 78°). The inventors have found that the mean value of the valgus angle in the studied population corresponded to a value of alpha prime of around 75°. Accordingly, the exchangeable liner component (132, 132') producing a valgus angle within a range encompassing the mean range provide a single best-fit solution to early implant wear and loosening.

The kit may comprise at least two exchangeable liner components (132, 132') each producing a different value of alpha prime, one producing a value alpha prime 86°-91°, the other producing a value alpha prime 68°-81°. The kit may comprise at least three exchangeable liner components (132, 132') each producing a different value of alpha prime, one producing a value alpha prime 61°-71°, the other producing a value alpha prime of 71°-81°, and the other producing a value alpha prime 81°-91°.

The kit may further comprise one or more humeral components (50), wherein each humeral component (50) comprises:
- a humeral stem portion (60) configured for placement within the humeral intramedullary canal, and
- the proximal part (130a) of the implant elbow joint (130).

The above-mentioned parts of the kit humeral component (50) including humeral stem portion (60), ulnar component (110) (without the angulation joint (140)), ulnar stem portion (120), bridging section (130) are as described elsewhere herein including constituting parts, components and variations. It is appreciated that, while the exchangeable liner component (132, 132') has been described as attaching to the distal part (130b) of the implant elbow joint (130), the same effects may be achieved by providing an exchangeable liner component (132, 132') for attachment the proximal part (130a) of the implant elbow joint (130). Accordingly, the parts of the description referring to the exchangeable liner component (132, 132') configured for dismountable attachment to the distal part (130b, 130b') of the elbow joint (130), apply *mutatis mutandis* to an exchangeable liner component (132, 132') configured for dismountable attachment to the proximal part (130a) of the elbow joint (130).

The humeral component (50) may be a monoblock. The monoblock is a one-piece element, typically formed by a process such as moulding, milling from a solid block, or additive manufacturing. The monoblock is rigid, meaning that it does not exhibit flexibility. More specifically, the humeral component (50) may be a monoblock with the proximal part (130a) of the implant elbow joint (130).

Alternatively, the humeral component (50) further comprises a humeral angulation joint (70) having at least one degree of freedom of rotational movement configured to adjust a direction of the humeral stem portion (60). An example of a humeral component (50) containing a humeral angulation joint (70) is shown in **FIG. 29****.** The humeral angulation joint (70) is a mechanical rotational joint having at least 1DOF of rotational movement (e.g. 1, 2 or 3 DOF). It is separate from the implant elbow joint (130). It may be configured to adjust a direction of the humeral stem portion (60) to correspond at least with a native direction of the humerus of the subject. The humeral angulation joint (70) may comprise a proximal humeral angulation coupling (70a) configured to couple to a distal humeral angulation coupling (70b), thereby forming the humeral angulation joint (70). The rotational direction of the humeral angulation joint (70) is lockable *i.e.* rotation of the humeral angulation joint can be prevented. The elbow joint prosthetic implant (100) may be provided with a humeral locking element (*e.g.* a fixation screw) (74) configured for locking a direction and/or axial rotational angle of the humeral stem portion (60). The humeral angulation joint may be provided without a locking element *i.e.* the humeral angulation joint may be free moving; the direction may be fixed during implantation by cement used to secure the humeral component (50) within the humeral intramedullary canal of the subject.

The direction of the humeral stem portion (60) of the implant refers to a direction of the humeral stem portion (60). The direction may be in one or more of varus (Vr), valgus (VI), anterior (A), posterior (P) directions. The direction may be in one or more of varus (Vr), valgus (VI), and optionally additionally in one or more of the anterior (A) and posterior (P) directions. Hence the humeral stem portion (60) may be adjustable in the aforementioned direction. It is referenced to an axis of rotation (A-A') of the implant elbow joint (130). Prior or during implantation, the humeral angulation joint (70) is adjusted and fixed so that the native direction of the flexion-extension axis (a-a') of the subject is restored.

The result is a restoration of native elbow kinematics, which reduces complications such as loosening and lining (*e.g.* polymer) wear. There is further a decrease in amount of revision surgery as loosening and wear are less frequent. There is further improved proprioception and stability after arthroplasty as the muscle tension between agonists and antagonists is in balance. The same implant can be applied to a wide population range.

The humeral angulation joint (70) may have only (exactly) 1DOF of rotational movement (around C-C'). The 1DOF of rotational movement of the humeral angulation joint (70) has an axis of rotation (C-C') that may be perpendicular to an axis of rotation (A-A') of the implant elbow joint (130). The axis of rotation (C-C') may be perpendicular ± 0 to 15° in any direction to an axis of rotation (A-A') of the implant elbow joint (130). The axis of rotation (C-C') of the humeral angulation joint (70) may or may not intersect the axis of rotation (A-A') of the implant elbow joint (130).

Examples of 1DOF of rotational movement is a revolute joint, in which the proximal humeral angulation coupling (70a) and the distal humeral angulation coupling (70b) are joined by fixture providing a single axis of rotation such as a pin, or nut and bolt or contains a machine taper.

The humeral angulation joint (70) may have exactly two or exactly three degrees of freedom of (2DOF or 3DOF) rotational movement, as exemplified in **FIG. 29****.** Such the implant (100) may be configured to adjust and fix a direction of the humeral stem portion (60) to correspond with a native direction of the humerus of a subject. One of the degrees of freedom preferably includes the axis of rotation (C-C') of the humeral angulation joint (70). The additional degrees of freedom allow movement in the anterior and/or posterior direction; additional rotation is clinically important. The 2DOF or 3DOF humeral angulation joint (70) comprises a ball joint. Advantageously, 2DOF or 3DOF humeral angulation joint (70) allow more directions for adjustment after placement; it is more convenient for the user to correct for imprecise implantation. The range of motion of the 2DOF or 3DOF humeral angulation joint (70) may be centred around the axis C-C', which corresponds to the 1DOF of rotational movement when the humeral angulation joint (70) has only 1DOF.

The humeral component (110) may comprise a humeral linking body (76) having a proximal end (10) and a distal end (20). The humeral linking body (76) is shown in enlarged view in **FIG. 30B****.** The distal end (20) of the humeral linking body (76) is disposed with the elbow joint proximal part (130a) described earlier. The proximal end (10) of the humeral linking body (76) is disposed with the distal humeral angulation coupling (70b) described earlier. A position and orientation of the elbow joint proximal part (130a) with respect to the distal humeral angulation coupling (70b) is fixed. A plurality of humeral linking bodies (76) may be provided, each having a different fixed position and/or fixed orientation between the elbow joint proximal part (130a) and the distal humeral angulation coupling (70b).

The linking body (76) is dismountable from a remainder of the humeral component (50). In other words, the humeral linking body (76) distal humeral angulation coupling (70b) is dismountable from the proximal humeral angulation coupling (70a) (see **FIG. 30A****).** The humeral linking body (76) is dismountable from a remainder of the implant (100). In other words, the humeral linking body (76) distal humeral angulation coupling (70b) is dismountable from the proximal humeral angulation coupling (70a), and the humeral linking body (76) elbow joint proximal part (130a) may be dismountable from the elbow joint distal part (130b).

A plurality of humeral linking bodies (76) may be provided, at least two preferably all having a different fixed position and/or different fixed orientation relation between the elbow joint distal part (130b) and the distal humeral angulation coupling (70b). The dismountability allows a humeral linking body (76) to be selected having a desired fixed position and/or fixed orientation relation between the elbow joint distal part (130b) and the distal humeral angulation coupling (70b). This allows for a displacement adjustment in a medio-lateral and/or anteroposterior direction and/or in cranio-caudal The plurality may be provided as a kit optionally with the implant.

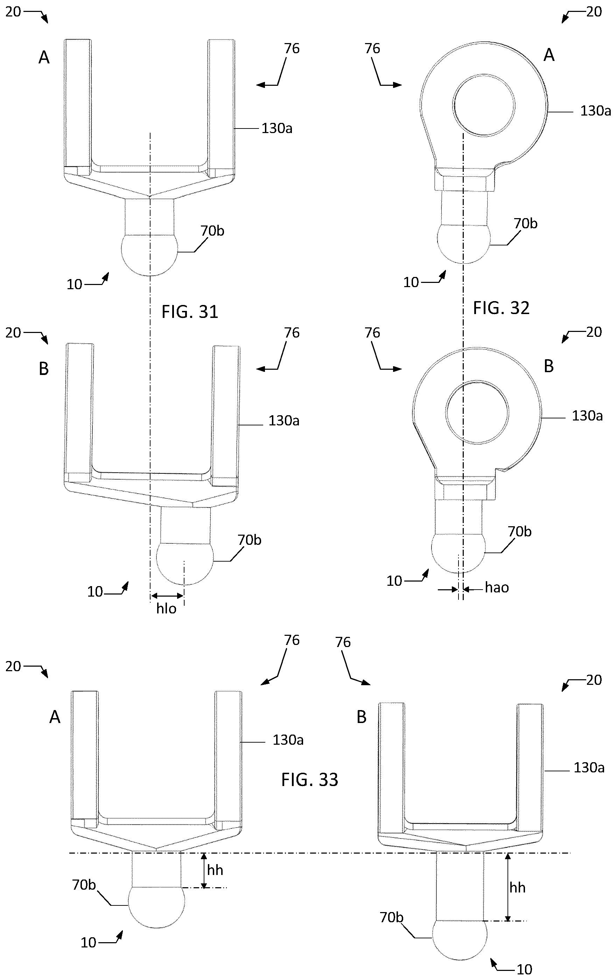

A plurality of humeral linking bodies (76) may be provided, at least two preferably all having a different fixed mediolateral offset (hlo) (displacement in a mediolateral direction) between the elbow joint distal part (130b) and the distal humeral angulation coupling (70b). The range of hlo includes zero. An example of a humeral linking body (76) with no offset is shown in **FIG. 31A****,** and with a mediolateral offset (hlo) is shown in **FIG. 31B****.** The dismountability allows a humeral linking body (76) to be selected having a desired mediolateral offset, allowing for a displacement adjustment in a mediolateral direction. The offset may be a mutual offset (*e.g.* with respect two or more humeral linking bodies (76)), with respect a neutral position of the distal humeral angulation coupling (70b) *e.g.* a central position of the distal humeral angulation coupling (70b), of with respect to another positional reference. The plurality may be provided as a kit optionally with the implant.

A plurality of humeral linking bodies (76) may be provided, at least two preferably all having a different fixed anteroposterior offset (hao) (displacement in an anteroposterior direction) between the elbow joint distal part (130b) and the distal humeral angulation coupling (70b). The range of hao includes zero. An example of a humeral linking body (76) with no offset is shown in **FIG. 32A****,** and with an anteroposterior (hao) is shown in **FIG. 32B****.** The dismountability allows a humeral linking body (76) to be selected having a desired anteroposterior offset, allowing for a displacement adjustment in an anteroposterior direction. The offset may be a mutual offset (*e.g.* with respect two or more humeral linking bodies (76)), with respect a neutral position of the distal humeral angulation coupling (70b) *e.g.* a central position of the distal humeral angulation coupling (70b), of with respect to another positional reference. The plurality may be provided as a kit optionally with the implant.

A plurality of humeral linking bodies (76) may be provided, at least two preferably all having a different fixed distance (hh) between the elbow joint distal part (130b) and the distal humeral angulation coupling (70b). The fixed distance (hh) provides different degrees of cranio-caudal translation. Examples of a humeral linking bodies (76) with different distances hh are shown in **FIG. 33A** and **FIG. 33B.** The dismountability allows a humeral linking body (76) to be selected having a desired distance (hh), allowing for a displacement adjustment in an anteroposterior direction. The plurality may be provided as a kit optionally with the implant.

A plurality of humeral linking bodies (76) may be provided, at least two preferably all having a different fixed angle between the elbow joint distal part (130b) and the distal humeral angulation coupling (70b). The dismountability allows a humeral linking body (76) to be selected having a desired angulation, allowing for an angulation adjustment in a varus/valgus (medio-lateral direction. The plurality may be provided as a kit optionally with the implant.

A plurality of humeral linking bodies (76) may be provided each having one or more (*e.g.* 2 or more) of the aforementioned offsets, distances or fixed angles.

The dismountability may be before, during or after placement *in situ* of implant (100). For example, when both (humeral and ulnar) components are cemented in the bone, the user could still change the humeral linking body (76) if the extension or flexion of the elbow still needs correcting. It also allows the humeral linking body (76) to be changed in revision surgery.

The humeral linking body (76) may be made from any biocompatible material having the requisite strength characteristic for an elbow joint prosthetic implant (100), such as one or more of titanium, stainless steel, tantalum, vanadium, cobalt, chromium, tungsten, nickel, molybdenum. The humeral linking body (76) is preferably formed from a mono-block *i.e.* is a one-piece element. This provides adjustability to the implant while still maintaining mechanical strength by avoiding a prismatic joint.

A fixing element (*e.g.* threaded member) may be provided configured for fixing the humeral linking body (78) to the remainder of the humeral stem portion (60) to prevent dismounting. The aforementioned locking element and fixing element may one and the same (*e.g.* threaded member).

Provided herein is a kit comprising:
- an ulnar component (110) (with or without ulnar angulation joint (140)),
- the humeral component (50) comprising the humeral angulation joint (70) and adapted for demountable attachment to a humeral linking body (76)
- a plurality of dismountable linking bodies (76), each having a different distance (hh), and
- the implant elbow joint (130) connecting the humeral component (50) and ulnar component (110).

The removeable humeral linking body (76) may be configured to provide the humeral angulation joint (70) with one, preferably only one degree of rotational freedom (around C-C' axis). In other words, the proximal humeral angulation coupling (70a) and distal humeral angulation coupling (70b) forming the humeral angulation joint (70) have one, preferably only one, degree of rotational freedom. One of the proximal humeral angulation coupling (70a) or distal humeral angulation coupling (70b) may comprise a tapered member, and the other of the proximal humeral angulation coupling (70a) or distal humeral angulation coupling (70b) comprises a reciprocating tapered hole configured to engage the tapered member thereby forming the one degree of rotational freedom humeral angulation joint (70).

The removeable humeral linking body (76) may be configured to provide the humeral angulation joint (70) with two or three, preferably only two or three, degrees of rotational freedom. In other words, the proximal humeral angulation coupling (70a) and distal humeral angulation coupling (70b) form the humeral angulation joint (70) having two or three, preferably only two or three, degrees of rotational freedom. A combination of a rotation around the medio-lateral axis and another distance (hh) allows a cranio-caudal and/or antero-posterior adjustment. One of the proximal humeral angulation coupling (70a) or distal humeral angulation coupling (70b) may comprises a spherical member (79), and the other of proximal humeral angulation coupling (70a) or distal humeral angulation coupling (70b) may comprises a reciprocating socket (77) configured to engage the spherical member (79), thereby forming the humeral angulation joint (70). The spherical member (79) may be part or all of a sphere. An example of a 2 or 3DOF removeable humeral linking body (76) is shown in **FIGs. 30B****.** The humeral linking body (76) has a different fixed distance (hh) between the elbow joint distal part (70b) and the proximal angulation coupling (70a).

The implant (100) described herein may be provided with an ulnar component (110) having ulnar angulation joint (140) and a humeral component (50) that is a monoblock (non-adjustable). The implant (100) described herein may be provided with an ulnar component (110) that is a monoblock (non-adjustable) and a humeral component (50) having humeral angulation joint (70). The implant (100) described herein may be with an ulnar component (110) having ulnar angulation joint (140) and a humeral component (50) having humeral angulation joint (70).

Provided herein is a measurement tool (600), not according to the claimed invention, for measurement of the direction of the ulna, in particular, of the proximal ulna. It may also be known as an ulnar measurement tool (600). It is principally a mechanical measurement tool, having parts for measurement of one or more angles. The measurement tool is similar to the elbow implant (100) in which the direction adopted by the angulation joint (140) is readable. It may be readable from a built-in scale, or built in sensor (*e.g.* rotary encoder) or by reading using a reader the angles adopted by the tool ulnar component (1110). The reader may be a device for measurement of one or more angle, and may include a readable scale or rotary encoder. An exemplary measurement tool (600) is shown in FIGs. **22A** and **22B****.**

According to one aspect, provided is a measurement tool (600) for determining a direction of an ulna (200) in particular of a proximal ulna (200a) of a subject, the measurement tool comprising:
- a tool humeral component (150) comprising a tool humeral stem portion (1160) configured for dismountable placement within a humeral intramedullary canal of the subject,
- a tool ulnar component (1110) comprising a tool ulnar stem portion (1120) configured for dismountable placement within the ulnar intramedullary canal (210) of the subject,
wherein
- the tool humeral component (150) and tool ulnar component (1110) are connected by a tool elbow joint (1130);
- the tool ulnar component (1110) further comprises a tool angulation joint (1140) configured to adjust a direction of the tool ulnar stem portion (1120),
- one or more angles adopted by tool angulation joint (1140) is readable.

By dismountable placement, it is meant that the humeral stem portion (160) or ulnar stem portion (1120) are removeable, after mounting therein, from the respective canal of the subject. Once the measurement is taken, the humeral stem portion (160) or ulnar stem portion (1120) can be removed from the from the respective canal of the subject. The mounting is preferably a fitting placement with minimum of play.

The above-mentioned parts of the tool: tool humeral component (150), tool humeral stem portion (160), tool ulnar component (1110), tool ulnar stem portion (1120), tool elbow joint (1130), tool angulation joint (1140) correspond to the implant parts: humeral component (50), humeral stem portion (60), ulnar component (110), ulnar stem portion (120), elbow revolute joint (130); angulation joint (140), respectively as described elsewhere herein, including constituting parts, components and variations. The tool angulation joint (1140) may also be known as an tool ulnar angulation joint (1140).

The tool humeral component (150) may be a monoblock or may contain a tool humeral angulation joint as described later below.

The tool ulnar component (1110) may further comprise a locking element; configured to lock the tool angulation joint (1140) and, hence, the direction of the tool ulnar stem portion (1120).

The tool ulnar component (1110) further comprises a tool bridging section (1124) is configured to introduce an angulation to orient the tool ulnar stem portion (1120) in a superior-inferior direction when the tool elbow joint (1130) is in extension. The tool bridging section (1124) corresponds with the implant bridging section (124) described elsewhere herein.

The one or more angles angle read by the tool determine a direction of an ulnar stem portion (120) of an elbow joint prosthetic implant (100). The one or more angles angle read by the tool is indicative of the selection of or the adjustment to the elbow joint prothesis (100). The tool angulation joint (1140) may be disposed with at least one readable angular scale configured to indicate one or more angles adopted by tool angulation joint. Alternatively or in addition, the one or more angles adopted by tool angulation joint may be read by a reader.

The tool angulation joint (1140) may have only one degree of freedom of rotational movement (*e.g.* around B-B'), and is configured to adjust a direction of the tool ulnar stem portion (1120). Preferably the direction corresponds with a native varus-valgus direction of the ulna (200) of a subject.

The tool angulation joint (1140) may have (exactly) only two or three degrees of freedom of rotational movement (*e.g.* around B-B'), and is configured to adjust a direction of the tool ulnar stem portion (1120). Preferably the direction corresponds with a native direction of the ulna (200) of a subject, which may include the native varus-valgus direction of the ulna (200) of a subject.

The measurement tool (600) may incorporate one or more electronic angle sensors for (*e.g.* rotary encoders) for measurement of the tool elbow joint (1130) and/or tool angulation joint (1140). The measurement tool (600) may incorporate one or more actuators (e.g. electronic movement device, such as servo motor) and/or one or more electronic angle sensors for (*e.g.* rotary encoders) for applying and or measuring forces for soft tissue balancing. The measurement tool (600) may be connectable to a surgical navigation device for measuring the angulations.

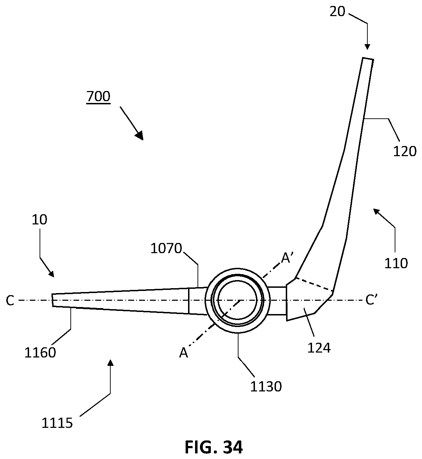

Provided herein is a humeral measurement tool (700), not according to the claimed invention, for measurement of the direction of the humerus. It is principally a mechanical measurement tool, having parts for measurement of one or more angles. The humeral measurement tool is similar to the elbow implant (100) in which the direction adopted by the humeral angulation joint (70) is readable. It may be readable from a built-in scale, or built in sensor (*e.g.* rotary encoder) or by reading using a reader the angles adopted by the tool humeral component (1115). The reader may be a device for measurement of one or more angle, and may include a readable scale or rotary encoder. An exemplary measurement tool (700) is shown in FIG. **34****.**

According to one aspect, provided is a humeral measurement tool (700) for determining a direction of a humerus of a subject, the humeral measurement tool (700) comprising:
- a tool ulnar component (110) comprising a tool ulnar stem portion (120) configured for dismountable placement within the ulnar intramedullary canal (210) of the subject,
- a tool humeral component (1115) comprising a tool humeral stem portion (1160) configured for dismountable placement within a humeral intramedullary canal of the subject,
wherein
- the tool humeral component (1115) and ulnar component (110) are connected by a tool elbow joint (1130);
- the tool humeral component (1115) further comprises a tool humeral angulation joint (1070) configured to adjust a direction of the tool humeral stem portion (1160),
- one or more angles adopted by tool humeral angulation joint (1070) is readable.

The above-mentioned parts of the tool: tool humeral component (1115), tool humeral stem portion (1160), tool ulnar component (1110), tool ulnar stem portion (120), tool elbow joint (1130), tool humeral angulation joint (1070) correspond to the implant parts: humeral component (50), humeral stem portion (60), ulnar component (110), ulnar stem portion (120), elbow revolute joint (130); humeral angulation joint (140), respectively as described elsewhere herein, including constituting parts, components and variations. The tool ulnar component (1110) may be a monoblock or may comprise an ulnar angulation joint (1140) as described elsewhere.

The tool humeral component (1115) may further comprise a locking element; configured to lock the tool humeral angulation joint (1070) and, hence, the direction of the tool humeral stem portion (1160).

The one or more angles read by the tool (700) may determine a direction of the humeral stem portion (1160) of an elbow joint prosthetic implant (100). The one or more angles angle read by the tool (700) may be indicative of the selection of or the adjustment to the elbow joint prothesis (100). The tool humeral angulation joint (1070) may be disposed with at least one readable angular scale configured to indicate one or more angles adopted by tool angulation joint. Alternatively or in addition, the one or more angles adopted by tool angulation joint may be read by a reader.

The tool humeral angulation joint (1070) may have only one degree of freedom of rotational movement (*e.g.* around C-C'), and is configured to adjust a direction of the tool humeral stem portion (1120). Preferably the direction corresponds with a native varus-valgus direction of the ulna (200) of a subject.

The tool humeral angulation joint (1070) may have (exactly) only two or three degrees of freedom of rotational movement (*e.g.* around C-C'), and is configured to adjust a direction of the tool humeral stem portion (1160). Preferably the direction corresponds with a native direction of the ulna (200) of a subject, which may include the native varus-valgus direction of the ulna (200) of a subject.

The humeral measurement tool (700) may incorporate one or more electronic angle sensors for (*e.g.* rotary encoders) for measurement of the tool elbow joint (1130) and/or tool humeral angulation joint (1070). The measurement tool (1100) may incorporate one or more actuators (*e.g.* electronic movement device, such as servo motor) and/or one or more electronic angle sensors for (*e.g.* rotary encoders) for applying and or measuring forces for soft tissue balancing. The measurement tool (700) may be connectable to a surgical navigation device for measuring the angulations.

The measurement tool (600) described herein may be provided with a tool ulnar component (1110) having tool ulnar angulation joint (1140) and a humeral component (150) that is a monoblock (non-adjustable). The measurement tool (700) described herein herein may be provided with an ulnar component (110) that is a monoblock (non-adjustable) and a humeral component (1115) having humeral angulation joint (1070). The measurement tool (700) described herein may be provided with an ulnar component (1110) having ulnar angulation joint (1140) and a humeral component (1115) having humeral angulation joint (1070).

A major problem in elbow arthroplasty is tendon-to-bone healing of the triceps. The triceps are often damaged or cut, depending on the procedure. The triceps-off approach reveals complication rates that are 4 times higher than the triceps-on approach. The main complication of triceps-off approach is triceps ruptures. An alternative technique is a triceps-on approach (without detaching the triceps tendon) by making a standardized olecranon osteotomy. After implantation of the implant, the olecranon is fixated to the ulna with a fixation system integrated in the implant itself.

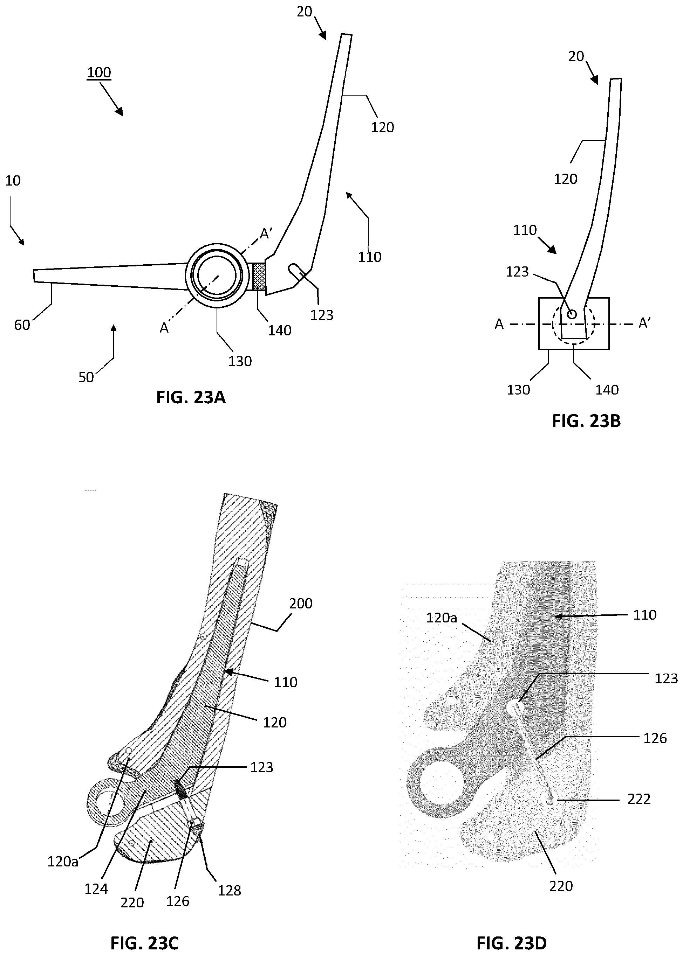

The fixation system comprises a fixation element receiver (123) disposed on the ulnar component (110) and a fixation element (126). The fixation element receiver (123) is configured to engage with (and secure) a fixation element (126). An example of the implant (100) provided with a fixation element receiver (123) is shown in **FIGs. 23A** to **23C****.**

The fixation element receiver (123) is configured such that it is disposed adjacent to the olecranon (220). The fixation element receiver (123) may be disposed on the proximal ulnar stem (120a). The fixation element receiver (123) may be disposed on the bridging section (124). The fixation element (126) may be a threaded bolt or screw and the fixation element receiver (123) may be a threaded hole. The fixation element (126) may be a suture or wire and the fixation element receiver (123) may be an anchoring post or hole configured to receive the suture.

The fixation element receiver (123) and fixation element (126) are configured for attachment of the olecranon (220) to the ulna (200). **FIG. 23C** shows a fixation element threaded hole (123) and fixation element (126) is threaded bolt or screw (126). The bolt or screw (126) shaft passes through the sectioned olecranon (220) thereby holding it in position for bone healing. A protective cap (128) is positioned over the bolt or screw (126) head. **FIG. 23D** shows a fixation element suture hole (123) and fixation element (126) is section of wire (126). The wire (126) shaft passes through a hole (222) in the sectioned olecranon (220) thereby holding it in position for bone healing.

### Experimental data

### Study subjects

Twenty-four healthy volunteers (Mean age 27.65 y (SD: 9.25 y); 20 males, 4 females) were recruited for a study. Volunteers were excluded if one of the following exclusion criteria were present: age < 18 years, history of upper extremity pathology/surgery and chronic disease. Two orthopedic surgeons confirmed the absence of any clinical and radiological abnormality.

### Ethics

The ethics committee of the Ghent University Hospital approved the study (reference B670201524945); written informed consent was obtained from all participants.

### CT-scan processing

The CT images were acquired using a Siemens SOMTOM Definition AS (Siemens, München, Germany). The voxel size was 0.45mm; 0.45 mm; 0.6 mm (slice thickness). Only the left elbow was scanned. First, the scans were segmented with Mimics Research 20.0 (Materialise NV, Leuven, Belgium) to obtain 3D reconstructions of the ulnar canal, ulna and humerus. Subsequent measurements were performed in 3-Matic Research 12.0 (Materialise NV, Leuven, Belgium).

### Measurements

Several measurements were performed in 3-Matic. The valgus angle of the ulnar intramedullary canal - the angle of measurement defining the proximal ulna intramedullary canal in a varus-valgus direction - was measured for each subject

### Statistics

Statistical analyses were performed with IBM SPSS 24 Statistics (IBM, New York, USA) and Microsoft Excel (Microsoft, Redmond, USA). The Pearson correlation coefficient was calculated to assess the correlation between the different measurements. The R² value was calculated to determine the linearity of the centerline in the frontal plane

### Results

A large valgus angle variation of the proximal canal in relation to the axis of rotation in this study population was. The mean valgus angulation of the proximal canal until the point of varus angulation is 15.28° (SD: 4.05°) and ranged between 7.38° and 21.01°. In terms of angle alpha, the mean value was 74.72° (SD: 4.05°) and ranged between 68.99° and 82.62°. In males, the mean variation was 15.92° (SD: 3.94°) whereas in females, the mean variation was 12.12° (SD 3.41°). The large range in the population is not accommodated by tradition prosthetic elbow implants having a fixed valgus angle.

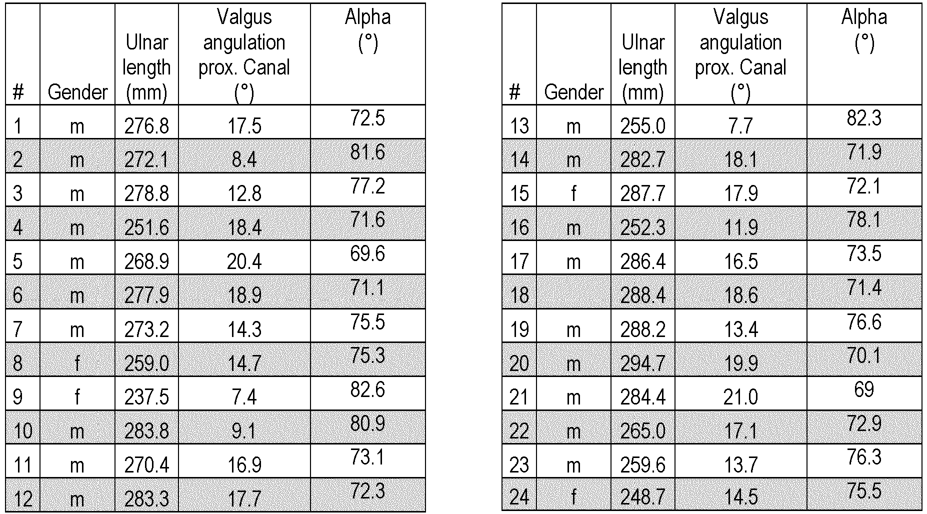

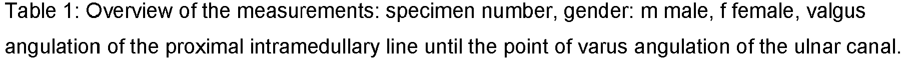

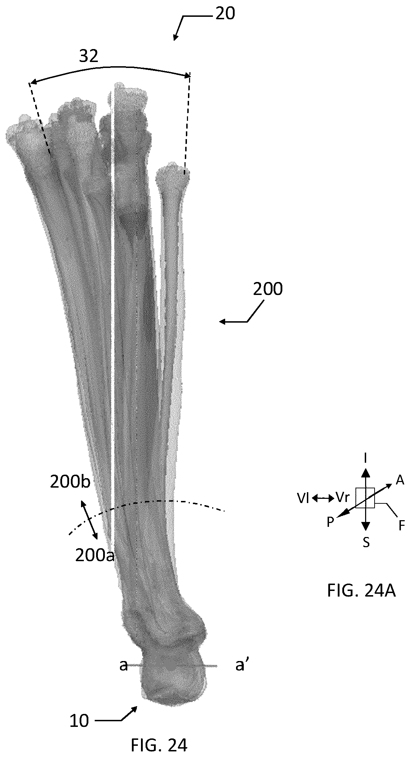

**FIG. 24** is a view of the imaged ulna superimposed with their proximal (10) joints aligned to demonstrate the broad range of valgus angulation of the proximal ulna that is amplified along the shaft to each distal (20) joint showing a wide spread (32) of positions.

## Claims

1. An elbow joint prosthetic implant (100) comprising:
- a humeral component (50) comprising a humeral stem portion (60) configured for placement within the humeral intramedullary canal of a subject,
- an ulnar component (110) comprising an ulnar stem portion (120) configured for placement within the ulnar intramedullary canal of the subject,
wherein the humeral component (50) and ulnar component (110) are connected by an implant elbow joint (130),
- wherein the ulnar component (110) further comprises an angulation joint (140) that is separate from the implant elbow joint (130), wherein the angulation joint (140) has at least one degree of freedom of rotational movement,
**characterised in that**:
- the angulation joint (140) is configured to adjust and fix a varus-valgus direction of the ulnar stem portion (120).

2. The elbow joint prosthetic implant (100) according to claim 1, wherein:
- the implant elbow joint (130) comprises a proximal part (130a) configured to couple to a distal part (130b) thereby forming the implant elbow joint (130);
- the angulation joint (140) comprises a proximal angulation coupling (140a) configured to couple to a distal angulation coupling (140b), thereby forming the angulation joint (140);
- the ulnar component (110) comprises a linking body (146) having a proximal end (10) and a distal end (20);
- the proximal end (10) of the linking body (146) is disposed with the distal part (130b) of the elbow joint (130);
- the distal end (20) of the linking body (146) is disposed with the proximal part (140a) of the angulation joint (140)
- a position and/or orientation of the distal elbow coupling (130b) and the proximal angulation coupling (140a) is fixed, and
- the linking body (146) is dismountable from the ulnar component (110).

3. The elbow joint prosthetic implant (100) according to claim 2, provided with a plurality of linking bodies (146), at least two, preferably all having a different fixed position and/or different fixed orientation relation between the elbow joint distal part (130b) and the proximal angulation coupling (140a), preferably wherein at least two preferably all of plurality of linking bodies (146) has a different fixed distance (h) between the elbow joint distal part (130b) and the proximal angulation coupling (140a).

4. The elbow joint prosthetic implant (100) according to claim 2 or 3, provided with:
- a locking element (148) configured for locking a direction and/or axial rotational angle of the ulnar stem portion (120), or
- a fixing element (148') configured for fixing the linking body (146) to the remainder of the ulnar stem portion (120), or
- a combined locking and fixing element (148, 148') configured for both locking a direction and/or axial rotational angle of the ulnar stem portion (120) and fixing the linking body (146) to the remainder of the ulnar stem portion (120).

5. The elbow joint prosthetic implant (100) according to any one of claims 2 to 4, wherein:
- the proximal angulation coupling (140a) and distal angulation coupling (140b) form the angulation joint (140) having one degree of rotational freedom,
- one of the proximal angulation coupling (140a) or distal angulation coupling (140b) comprises a tapered member (142a), and
- the other of the proximal angulation coupling (140a) or distal angulation coupling (140b) comprises a reciprocating tapered hole (142b) configured to engage the tapered member (142a) thereby forming the one degree of rotational freedom angulation joint (140).

6. The elbow joint prosthetic implant (100) according to any one of claims 2 to 4, wherein:
- the proximal angulation coupling (140a) and distal angulation coupling (140b) form the angulation joint (140) having two or three degrees of rotational freedom,
- one of the proximal angulation coupling (140a) or distal angulation coupling (140b) comprises a spherical member (144a), and
- the other of proximal angulation coupling (140a) or distal angulation coupling (140b) comprises a reciprocating socket (144b) configured to engage the spherical member (144a), thereby forming the two or three degrees of rotational freedom angulation joint (140).

7. The elbow joint prosthetic implant (100) according to any of claims 1 to 6, wherein the ulnar stem portion (120) is a rigid monoblock.

8. The elbow joint prosthetic implant (100) according to any one of claim 1 to 6, wherein the ulnar stem portion (120) is at least partially articulated, allowing the ulnar stem portion (120) to conform to the shape of the ulnar intramedullary canal (210).

9. A kit (500) comprising:
- a humeral component (50) comprising a humeral stem portion (60) configured for placement within the humeral intramedullary canal of a subject,
- at least two ulnar components (110, 110') each being a monoblock, wherein each ulnar component (110, 110') comprises:
∘ an ulnar stem portion (120) configured for placement within the ulnar intramedullary canal (210) of the subject, and
∘ a distal part (130b, 130b') of an implant elbow joint (130), configured to engage with a corresponding proximal part (130a) of the implant elbow joint (130) of the humeral component (50), thereby forming the implant elbow joint (130),
**characterised in that**:
at least two of the ulnar components (110, 110') mutually differ in a valgus-varus direction of the ulnar stem portion (120), and
at least one of the ulnar components (110, 110') has a valgus angle of between -1° and 4° or between 9° and 29°.

10. The kit according to claim 9, wherein the at least one ulnar component (110, 110') has a valgus angle between 9° and 29°, preferably between 10° and 20°, more preferably between 12° and 18°.

## Patentansprüche

1. Prothetisches Ellenbogengelenkimplantat (100), umfassend:
ein Humerusbauteil (50), umfassend einen Humerusstielabschnitt (60), konfiguriert zur Platzierung innerhalb des humeralen Knochenmarkskanals eines Subjekts,
ein Ulnarbauteil (110), umfassend einen Ulnarstielabschnitt (120), konfiguriert zur Platzierung innerhalb des ulnaren Knochenmarkskanals des Subjekts,
wobei das Humerusbauteil (50) und das Ulnarbauteil (110) durch ein Implantat-Ellbogengelenk (130) verbunden sind,
wobei das Ulnarbauteil (110) ferner ein Angulationsgelenk (140) umfasst, das vom Implantat-Ellbogengelenk (130) getrennt ist, wobei das Angulationsgelenk (140) wenigstens einen Rotationsfreiheitsgrad aufweist,
**dadurch gekennzeichnet, dass**:
das Angulationsgelenk (140) konfiguriert ist, um eine Varus-Valgus-Richtung des Ulnarstielabschnitts (120) anzupassen und zu fixieren.

2. Prothetisches Ellenbogengelenkimplantat (100) nach Anspruch 1, wobei:
das Implantat-Ellbogengelenk (130) ein proximales Teil (130a) umfasst, das konfiguriert ist, um mit einem distalen Teil (130b) gekoppelt zu werden, wodurch das Implantat-Ellbogengelenk (130) gebildet wird;
das Angulationsgelenk (140) eine proximale Angulationskopplung (140a) umfasst, die konfiguriert ist, um mit einer distalen Angulationskopplung (140b) gekoppelt zu werden, wodurch das Angulationsgelenk (140) gebildet wird;
das Ulnarbauteil (110) einen Verbindungskörper (146) umfasst, der ein proximales Ende (10) und ein distales Ende (20) aufweist; das proximale Ende (10) des Verbindungskörpers (146) mit dem distalen Teil (130b) des Ellbogengelenks (130) angeordnet ist;
das distale Ende (20) des Verbindungskörpers (146) mit dem proximalen Teil (140a) des Angulationsgelenks (140) angeordnet ist
eine Position und/oder Orientierung der distalen Ellbogenkopplung (130b) und der proximalen Angulationskopplung (140a) fixiert ist, und
der Verbindungskörper (146) vom Ulnarbauteil (110) demontierbar ist.

3. Prothetisches Ellenbogengelenkimplantat (100) nach Anspruch 2, bereitgestellt mit einer Vielzahl von Verbindungskörpern (146), wenigstens zwei, vorzugsweise alle, die eine unterschiedliche feste Position und/oder unterschiedliche feste Orientierungsbeziehung zwischen dem distalen Ellbogengelenkteil (130b) und der proximalen Angulationskopplung (140a) aufweisen, wobei vorzugsweise wenigstens zwei, vorzugsweise alle, der Vielzahl von Verbindungskörpern (146) einen unterschiedlichen festen Abstand (h) zwischen dem distalen Ellbogengelenkteil (130b) und der proximalen Angulationskopplung (140a) aufweisen.

4. Prothetisches Ellenbogengelenkimplantat (100) nach Anspruch 2 oder 3, bereitgestellt mit:
einem Verriegelungselement (148), das zum Verriegeln einer Richtung und/oder eines axialen Rotationswinkels des Ulnarstielabschnitts (120) konfiguriert ist, oder
einem Fixierungselement (148'), das zum Fixieren des Verbindungskörpers (146) am Rest des Ulnarstielabschnitts (120) konfiguriert ist, oder
einem kombinierten Verriegelungs- und Fixierungselement (148, 148'), das sowohl zum Verriegeln einer Richtung und/oder eines axialen Rotationswinkels des Ulnarstielabschnitts (120) als auch zum Fixieren des Verbindungskörpers (146) am Rest des Ulnarstielabschnitts (120) konfiguriert ist.

5. Prothetisches Ellenbogengelenkimplantat (100) gemäß einem der Ansprüche 2 bis 4, wobei:
die proximale Angulationskopplung (140a) und die distale Angulationskopplung (140b) das Angulationsgelenk (140) bilden, das einen Rotationsfreiheitsgrad aufweist,
eine der proximalen Angulationskopplung (140a) oder der distalen Angulationskopplung (140b) ein sich verjüngendes Element (142a) umfasst, und
die andere der proximalen Angulationskopplung (140a) oder der distalen Angulationskopplung (140b) ein sich verjüngendes Gegenloch (142b) umfasst, das konfiguriert ist, um das sich verjüngende Element (142a) in Eingriff zu nehmen, wodurch das Angulationsgelenk (140) mit einem Rotationsfreiheitsgrad gebildet wird.

6. Prothetisches Ellenbogengelenkimplantat (100) gemäß einem der Ansprüche 2 bis 4, wobei:
die proximale Angulationskopplung (140a) und die distale Angulationskopplung (140b) das Angulationsgelenk (140) bilden, das zwei oder drei Rotationsfreiheitsgrade aufweist,
eine der proximalen Angulationskopplung (140a) oder der distalen Angulationskopplung (140b) ein sphärisches Element (144a) umfasst, und
die andere der proximalen Angulationskopplung (140a) oder der distalen Angulationskopplung (140b) eine sphärische Gegenpassung (144b) umfasst, die konfiguriert ist, um das sphärische Element (144a) in Eingriff zu nehmen, wodurch das Angulationsgelenk (140) mit zwei oder drei Rotationsfreiheitsgraden gebildet wird.

7. Prothetisches Ellenbogengelenkimplantat (100) gemäß einem der Ansprüche 1 bis 6, wobei der Ulnarstielabschnitt (120) ein starrer Monoblock ist.

8. Prothetisches Ellenbogengelenkimplantat (100) gemäß einem der Ansprüche 1 bis 6, wobei der Ulnarstielabschnitt (120) wenigstens teilweise artikuliert ist, was es dem Ulnarstielabschnitt (120) ermöglicht, sich der Form des ulnaren Knochenmarkskanals (210) anzupassen.

9. Kit (500), umfassend:
ein Humerusbauteil (50), umfassend einen Humerusstielabschnitt (60), konfiguriert zur Platzierung innerhalb des humeralen Knochenmarkskanals eines Subjekts,
wenigstens zwei Ulnarbauteile (110, 110'), von denen jede ein Monoblock ist, wobei jedes Ulnarbauteil (110, 110') umfasst:
∘ einen Ulnarstielabschnitt (120), konfiguriert zur Platzierung innerhalb des ulnaren Knochenmarkskanals (210) des Subjekts, und
∘ ein distales Teil (130b, 130b') eines Implantat-Ellbogengelenks (130), konfiguriert zum Eingriff mit einem entsprechenden proximalen Teil (130a) des Implantat-Ellbogengelenks (130) des Humerusbauteils (50), wodurch das Implantat-Ellbogengelenk (130) gebildet wird,
**dadurch gekennzeichnet, dass**:
wenigstens zwei der Ulnarbauteile (110, 110') sich gegenseitig in einer Valgus-Varus-Richtung des Ulnarstielabschnitts (120) unterscheiden, und
wenigstens eins der Ulnarbauteile (110, 110') einen Valguswinkel zwischen -1° und 4° oder zwischen 9° und 29° aufweist.

10. Kit nach Anspruch 9, wobei die wenigstens ein Ulnarbauteil (110, 110') einen Valguswinkel zwischen 9° und 29° aufweist, vorzugsweise zwischen 10° und 20°, bevorzugter zwischen 12° und 18°.

## Revendications

1. Implant prothétique d'articulation du coude (100) comprenant :
- un composant huméral (50) comprenant une partie de tige humérale (60) configurée pour être placée dans le canal intramédullaire huméral d'un sujet,
- un composant cubital (110) comprenant une partie de tige cubitale (120) configurée pour être placée dans le canal intramédullaire cubital d'un sujet,
dans lequel le composant huméral (50) et le composant cubital (110) sont reliés par une articulation de coude d'implant (130),
- dans lequel le composant cubital (110) comprend également une articulation d'angulation (140) qui est séparée de l'articulation de coude d'implant (130), dans lequel l'articulation d'angulation (140) a au moins un degré de liberté de mouvement de rotation,
**caractérisé en ce que** :
- l'articulation d'angulation (140) est configurée pour ajuster et fixer une direction varus-valgus de la partie de tige cubitale (120).

2. Implant prothétique d'articulation du coude (100) selon la revendication 1, dans lequel :
- l'articulation de coude d'implant (130) comprend une partie proximale (130a) configurée pour se coupler à une partie distale (130b) formant ainsi l'articulation de coude d'implant (130) ;
- l'articulation d'angulation (140) comprend un couplage d'angulation proximal (140a) configuré pour se coupler à un couplage d'angulation distal (140b), formant ainsi l'articulation d'angulation (140) ;
- le composant cubital (110) comprend un corps de liaison (146) ayant une extrémité proximale (10) et une extrémité distale (20) ;
- l'extrémité proximale (10) du corps de liaison (146) est disposée avec la partie distale (130b) de l'articulation du coude (130) ;
- l'extrémité distale (20) du corps de liaison (146) est disposée avec la partie proximale (140a) de l'articulation d'angulation (140)
- une position et/ou une orientation du couplage du coude distal (130b) et du couplage d'angulation proximal (140a) est fixe, et
- le corps de liaison (146) est démontable du composant cubital (110).

3. Implant prothétique d'articulation du coude (100) selon la revendication 2, pourvu d'une pluralité de corps de liaison (146), au moins deux, de préférence tous ayant une position fixe différente et/ou une relation d'orientation fixe différente entre la partie distale de l'articulation du coude (130b) et le couplage d'angulation proximal (140a), de préférence dans lequel au moins deux, de préférence tous, de la pluralité de corps de liaison (146) ont une distance fixe différente (h) entre la partie distale de l'articulation du coude (130b) et le couplage d'angulation proximal (140a).

4. Implant prothétique d'articulation du coude (100) selon la revendication 2 ou 3, pourvu de :
- un élément de verrouillage (148) configuré pour verrouiller une direction et/ou un angle de rotation axial de la partie de tige cubitale (120), ou
- un élément de fixation (148') configuré pour fixer le corps de liaison (146) au reste de la partie de tige cubitale (120), ou
- un élément de verrouillage et de fixation combiné (148, 148') configuré pour verrouiller à la fois une direction et/ou un angle de rotation axial de la partie de tige cubitale (120) et fixer le corps de liaison (146) au reste de la partie de tige cubital (120).

5. Implant prothétique d'articulation du coude (100) selon l'une quelconque des revendications 2 à 4, dans lequel :
- le couplage d'angulation proximal (140a) et le couplage d'angulation distal (140b) forment l'articulation d'angulation (140) ayant un degré de liberté de rotation,
- l'un du couplage d'angulation proximal (140a) ou du couplage d'angulation distal (140b) comprend un élément conique (142a), et
- l'autre du couplage d'angulation proximal (140a) ou du couplage d'angulation distal (140b) comprend un trou conique complémentaire (142b) configuré pour engager l'élément conique (142a) formant ainsi le joint d'angulation à un degré de liberté de rotation (140).

6. Implant prothétique d'articulation du coude (100) selon l'une quelconque des revendications 2 à 4, dans lequel :
- le couplage d'angulation proximal (140a) et le couplage d'angulation distal (140b) forment l'articulation d'angulation (140) ayant deux ou trois degrés de liberté de rotation,
- l'un du couplage d'angulation proximal (140a) ou du couplage d'angulation distal (140b) comprend un élément sphérique (144a), et
- l'autre du couplage d'angulation proximal (140a) ou du couplage d'angulation distal (140b) comprend une douille complémentaire (144b) configurée pour engager l'élément sphérique (144a) formant ainsi le joint d'angulation à deux ou trois degrés de liberté de rotation (140).

7. Implant prothétique d'articulation du coude (100) selon l'une quelconque des revendications 1 à 6, dans lequel la partie de tige cubitale (120) est un monobloc rigide.

8. Implant prothétique d'articulation du coude (100) selon l'une quelconque des revendications 1 à 6, dans lequel la partie de tige cubitale (120) est au moins partiellement articulée, permettant à la partie de tige cubital (120) de se conformer à la forme du canal intramédullaire cubital (210).

9. Kit (500) comprenant :
- un composant huméral (50) comprenant une partie de tige humérale (60) configurée pour être placée dans le canal intramédullaire huméral d'un sujet,
- au moins deux composants cubitaux (110, 110') étant chacun un monobloc, dans lequel chaque composant cubital (110, 110') comprend :
∘ un composant de tige cubital (120) configuré pour être placée dans le canal intramédullaire cubital (210) du sujet, et
∘ une partie distale (130b, 130b') d'une articulation de coude d'implant (130), configurée pour s'engager avec une partie proximale correspondante (130a) de l'articulation de coude d'implant (130) du composant huméral (50), formant ainsi l'articulation de coude d'implant (130),
**caractérisé en ce que** :
- au moins deux des composants cubitaux (110, 110') diffèrent mutuellement dans une direction valgus-varus de la partie de tige cubital (120), et
- au moins un des composants cubitaux (110, 110') présente un angle de valgus compris entre -1° et 4° ou entre 9° et 29°.

10. Kit selon la revendication 9, dans lequel l'au moins un composant cubital (110, 110') présente un angle de valgus compris entre 9° et 29°, de préférence entre 10° et 20°, plus préférablement entre 12° et 18°.
